# FASCICULE DE BREVET EUROPEEN

(11) **EP 1 337 556 B1**
(45) Date de publication et mention de la délivrance du brevet: **27.03.2019**
(21) Numéro de dépôt: 01998559.7
(22) Date de dépôt: 29.11.2001
(51) Int. Cl.: C07K 14/47, C12N 5/18, C12N 15/12, C12N 15/63, C07K 16/30, A61K 39/385, A61K 39/395, G01N 33/68, A61P 35/02

(54) **PEPTIDES IMMUNOGENIQUES MUTES DERIVES DE R9M, POLYNUCLEOTIDES LES CODANT ET LEURS USAGES THERAPEUTIQUES**
DURCH MUTATION DES R9M ENTSTANDENE IMMUNOGENE PEPTIDE, FÜR SIE KODIERENDE POLYNUKLEOTIDE UND IHRE THERAPEUTISCHE NUTZUNG
MUTATED IMMUNOGENIC PEPTIDES DERIVED FROM R9M, POLYNUCLEOTIDES CODING FOR SAME AND THERAPEUTIC USES THEREOF

(30) Priorité: 01.12.2000 CA 2325666
(43) Date de publication de la demande: 27.08.2003
(73) Titulaire: INSTITUT PASTEUR, 75724 Paris Cedex 15 (FR); INSTITUT NATIONAL DE LA SANTE ET DE LA RECHERCHE MEDICALE (INSERM), 75654 Paris Cédex 13 (FR); ASSISTANCE PUBLIQUE, HOPITAUX DE PARIS, 75004 Paris (FR)
(72) Inventeur: FIRAT, Hüseyin, F-75013 Paris (FR); LANGLADE-DEMOYEN Pierre, F-75015 Paris (FR); VILMER, Etienne, F-75013 Paris (FR); LEMONNIER, François, F-92340 Bourg-la-Reine (FR); ROHRLICH, Pierre, F-94160 Saint Mande (FR); YOTNDA, Patricia, c/o Institut Pasteur, F-75724 Cedex 15 (FR)
(74) Mandataire: Desaix, Anne
(86) Numéro de dépôt international: PCT/FR2001/003779
(87) Numéro de publication internationale: WO 2002/044202

(56) Documents cités:
- YUN, C. ET AL: "Augmentation of immune response by altered peptide ligands of the antigenic peptide in a human CD4+ T-cell clone reacting to TEL/AML1 fusion protein" TISSUE ANTIGENS (1999), 54(2), 153-161 , août 1999 (1999-08), XP002216786
- YOTNDA, P. ET AL: "Cytotoxic T cell response against the chimeric ETV6-AML1 protein in childhood acute lymphoblastic leukemia" JOURNAL OF CLINICAL INVESTIGATION (1998), 102(2), 455-462 , 15 juillet 1998 (1998-07-15), XP002216787

## Description

### a) Domaine de l'invention

La présente invention se rapporte à l'optimisation du peptide R9M sauvage et à l'utilisation des peptides obtenus pour la vaccination thérapeutique et/ou la vaccination préventive de la leucémie chez l'homme. Plus particulièrement, la présente invention vise des peptides immunogéniques R9M mutés comme agents anti-tumoraux de la leucémie aiguë lymphoblastique (LAL).

### b) Brève description de l'art antérieur

La leucémie aiguë lymphoblastique (LAL) est le cancer le plus fréquent chez l'enfant. Environ 450 nouveaux cas sont traités chaque année en France. Malgré les progrès des protocoles de chimiothérapie, 20 à 30 % de ces enfants ont des formes graves résistantes au traitement conventionnel ou guérissant au prix de risque de toxicité sévère. Les progrès relatifs des greffes de cellules souches et l'accessibilité plus grande de donneurs non apparentés n'apportent que dans certains cas la guérison à ces patients. Dans ce contexte, la recherche d'alternatives thérapeutiques à la lumière des progrès de l'immunologie apparaît légitime. Les progrès de l'immunité antitumorale peuvent être appliqués au domaine des hémopathies malignes.

### Immunité antitumorale et antileucémique

L'identification des épitopes d'antigènes associés aux cellules tumorales a permis de développer de nouvelles approches immunothérapeutiques applicables aux pathologies tumorales humaines. De nombreux laboratoires se sont alors attachés à l'identification de peptides épitopiques tumoraux. Si le mélanome est la première illustration de l'intérêt de cette démarche, elle est maintenant étendue avec succès à d'autres pathologies tumorales: cancer du rein, de la vessie, du sein, de l'utérus, et plus récemment des leucémies [Robinet et coll. Hematother 7: 205-215, (1998); Rooney et coll., Lancet, 345:9-13, (1995)]. Des essais cliniques ont déjà été engagés, utilisant soit des peptides épitopiques synthétiques, soit des virus recombinants, avec des résultats encourageants même si, dans la plupart des cas, la caractérisation des réponses lymphocytaires T induites n'a pu être assurée.

L'existence d'une réponse immune anti-leucémique a été observée dans les greffes de cellules souches hématopoïétiques. En effet, les greffes autologues ou allogéniques déplétées en cellules T sont associées à de fréquentes rechutes. Par contre les greffes non T déplétées sont associées à un risque de réaction du greffon contre l'hôte (GVHD) plus élevé mais celui de rechute est plus faible: le greffon a donc un effet anti-leucémique (GVL : graft versus leukemia). Le rôle de la réponse immune dans les hémopathies malignes à été confirmé par l'effet thérapeutique remarquable mais inconstant des transfusions de lymphocytes du donneur en cas de rechute post greffe surtout dans les LMC. L'anergie induite par l'absence d'expression de B7 sur les cellules leucémiques pourrait expliquer que cet effet GVL est plus marqué pour les leucémies myéloïdes chroniques (LMC) que pour les LAM et les LAL. Néanmoins, plusieurs équipes, dont celle de la Demanderesse, a montré l'existence de CTL spécifiques contre les cellules leucémiques de type LAL de type B et LAM [Boyer et coll., Blood, 89:3477-85, (1997); Buzyn et coll., Europ. J. Immunol., 27:1066-72, (1997); Heslop et coll., Nat. Med. 2:551-555, (1996); Yotnda et coll., J. Clin. Invest. 101:2290-6, (1998); Yotnda et coll., J. Clin. Invest. 102:455-62, (1998)]. Ainsi, l'équipe de la Demanderesse a démontré que les translocations chromosomiques peuvent générer des néo-antigènes. Dans le cas de la LMC et de la LAL-B avec t(12;21), les produits de fusion BCR-ABL ou TEL-AML1 exprimés uniquement sur les cellules leucémiques induisent des CTL spécifiques.

### Les gènes TEL et AML1 et la translocation (p12 ;q21)

La translocation t(12;21) intéresse 25% des LAL de la lignée B chez l'enfant. Elle est normalement mise en évidence par des techniques moléculaires (RT-PCR) ou d'hybridation *in situ* (FISH). Les gènes TEL sur le chromosome 12 et AML1 sur le chromosome 21 sont impliqués dans cette translocation. Le gène TEL (Translocation ETS Leukemia) appartient à la famille des gènes ETS qui codent pour des facteurs de régulation de transcription [Fenrick et coll., Mol. Cell. Biol. 20:5828-39 (2000)]. Par leur domaine ETS, ces protéines se lient à l'ADN au niveau de séquences spécifiques [Guidez et coll., Blood 96:2557-61 (2000)]. Des études ont montré une perte allélique de TEL dans 15-40 % des cas de LAL de la lignée-B de l'enfant. Ce gène répresseur est également impliqué dans les translocations t(5;12), t(9;12) et t(12;21) impliquées dans les hémopathies malignes.

Le gène AML1 (Acute Myeloide Leukemia-1) appartient à une famille de gènes codant pour des facteurs de transcription caractérisés par la présence d'un domaine de fixation à l'ADN, le domaine *runt.* Le gène AML1 est composé de 9 exons. AML1 est exprimé dans tous les tissus à l'exception du coeur et du cerveau. Il coderait pour un facteur de transcription impliqué dans la différenciation de la lignée myéloïde. Ce gène est également associé aux translocations t(8;21) et t(3;21) rencontrées dans les leucémies aiguës myéloblastiques.

Le point de cassure de la translocation t(12;21) est localisé entre les exons 5 et 6 de TEL et dans les introns 1 ou 2 de AML1. Le transcrit de fusion TEL AML1 est toujours retrouvé dans l'ARN des cellules lymphoblastiques de patients porteurs de la translocation (12;21) mais jamais dans les tissus normaux. Ce transcrit est composé de la partie NH₂ terminale de TEL et de la presque totalité de AML1. Deux transcrits de longueur différente et dont l'extrémité 5' varie sont produits. La forme la plus longue est présente dans la majorité des cas de LAL. Le rôle de ces protéines de fusion dans la leucémogénèse reste encore à définir.

### Aspects cliniques

Les LAL de la lignée B exprimant le gène de fusion TEL/AML1 atteignent des enfants âgés de 1 à 10 ans ayant habituellement moins de 50 000 blastes au diagnostic, sans hyperdiploïdie à l'examen cytogénétique [Baruchel et coll., Br. J. Heamatol. 99:101-106 (1997); Ma et coll., Hematol. Oncol. 17:91-95 (1999)]. La présence du transcrit TEL/AML1 a été associé à une diminution du risque de rechute dans plusieurs études.

Ces études ne doivent pas masquer la relative hétérogénéité de ces LAL: différentes publications ainsi que l'expérience personnelle des présents inventeurs suggèrent qu'environ 15% des cas de LAL avec translocation (12;21) se caractérisent par leur très mauvais pronostic sans que les mécanismes responsables en soient connus [Seeger et coll., Blood 91:1716-22 (1998)]. Cette population d'enfants est particulièrement concernée par de futurs protocoles d'immunothérapie.

### Résultats du domaine public obtenus dans les laboratoires de la Demanderesse

### 1) Réponse T cytotoxique contre la protéine chimère codée par le gène de fusion TEL- [Yotnda et coll., J. Clin. Invest. 102:455-62 (1998)]

Yotnda et coll., J. Clin. Invest. 102:455-62 (1998) a décrit l'identification de l'épitope R9M et son implication dans la réponse T cytotoxique chez les patients atteints de leucémie lymphoblastique aiguë.

Le produit des gènes chimères issus de la translocation (12;21) est une néoprotéine qui est exprimée dans les cellules leucémiques de type LAL-B commun et qui peut être détectée par Western blot. La Demanderesse a postulé que des peptides issus de cette région de jonction pouvaient être des antigènes tumoraux reconnus par des effecteurs cytotoxiques. Dans un premier temps, la capacité de 9 nonapeptides jonctionnels d'inhiber la fixation sur des molécules HLA-A*0201 solubles a été mesurée. Un nonapeptide RIAECILGM (R9M) codé par cette région de fusion a été identifié qui présente une haute affinité pour la molécule HLA-A2 soluble.

Au diagnostic, avant tout traitement, chez un patient HLA A2 porteur de la translocation (12;21) une lignée lymphocytaire T-CD8⁺ a été établie à partir de la moelle en présence de cellules blastiques autologues et de la forme soluble du ligand de CD40. Ces lymphocytes T lysent spécifiquement la leucémie autologue et reconnaissent le peptide à la surface des cibles HLA-A*0201 chargées avec le peptide R9M. Des résultats similaires sont obtenus au niveau clonal. L'ensemble des données résultant de cette étude suggère que le peptide R9M de cette translocation est immunogène et induit *in vitro* chez les patients HLA-A*0201 des réponses T spécifiques.

Cependant, l'évolution naturelle de la maladie, malgré l'existence de cette réponse immune, montre que la réponse développée est insuffisante pour contrôler cette prolifération tumorale, ou que les cellules tumorales interfèrent négativement avec les processus d'activation et de mise en oeuvre de la réponse cytotoxique. La capacité de R9M d'induire des CTLs à partir du sang périphérique de donneurs sains a été démontrée. Ces CTL spécifiques sont capables de reconnaître des cibles HLA-A*0201 pré-incubées avec le peptide sélectionné et ils reconnaissent des cellules leucémiques exprimant la protéine de fusion TEL-AML1 issues de patients HLA A2.

### 2) Absence de réponse antitumorale efficace dans les LAL [Yotnda et coll., Exp. Hematol. 27:1375-83, (1999)]

L'expression de certaines molécules de co-stimulation a été étudiée à la surface des lymphoblastes. Dans la majorité des cas, la molécule B7.1 est absente de la surface des blastes mais est exprimée en présence de la forme soluble du ligand de CD40. Il apparaît donc vraisemblable que les signaux d'activation délivrés aux lymphocytes-T soient insuffisants [Dilloo et coll., Blood 90:1927-33 (1997)]. Cette conclusion est supportée par un certain nombre de données complémentaires tirées de l'étude directe des lymphocytes-T CD3⁺ médullaires au diagnostic. Ces lymphocytes ont des profils de sécrétion de cytokines de type Th2 et sont plus fréquemment en apoptose. Ce processus d'anergie des CTLs spécifiques a également été mis en évidence dans d'autres pathologies tumorales [Boyer et coll., Blood 89:3477-85 (1997); Dunussi-Joannopoulos et coll., Blood 89:2915-24 (1997)].

### Optimisation des peptides épitopiques de la région de fusion de TEL AML

Les inventeurs de la présente invention ont observé que le complexe R9M/HLA A2.01 à la surface des cellules T2 était peu stable. Des efforts répétés d'immunisation des souris transgéniques HLA A2.01 avec le peptide R9M ont aussi montré l'inconstance et la faiblesse des réponses de CTL induites. Il apparaît donc clairement que le peptide R9M doit être optimisé pour devenir un agent antitumoral efficace.

Il existe donc un besoin pour des peptides R9M mutés présentant une haute affinité pour la molécule HLA-A2.01 de manière à former avec cette molécule un complexe stable ayant une demi-vie supérieure à la demi-vie du complexe peptide-R9M sauvage/HLA-A2.01 afin de rendre le peptide capable d'induire *in vivo* ou *in vitro* une réponse immunitaire cytotoxique.

Il existe aussi un besoin pour des peptides R9M mutés qui peuvent ralentir le développement de cellules tumorales lymphoblastiques lorsque administrés à un patient leucémique.

Plus particulièrement, il existe un besoin pour des peptides R9M mutés capables d'induire une réponse immunitaire contre le développement de cellules tumorales lymphoblastiques après administration à un receveur.

La présente invention répond à ces besoins et à d'autres besoins comme cela sera apparent à une personne versée dans le domaine à la lecture de la présente description de l'invention.

### RÉSUMÉ DE L'INVENTION

La présente invention se rapporte à l'optimisation du peptide R9M sauvage et à l'utilisation des peptides obtenus à des fins de vaccination thérapeutique et/ou de vaccination préventive de la leucémie chez l'homme.

L'invention vise des peptides immunogéniques mutés dérivant de la protéine humaine de fusion TEL/AML1 comprenant la séquence peptidique R9M sauvage (Arg-Ile-Ala-Glu-Cys-Ile-Leu-Gly-Met), lesdits peptides étant mutés dans la séquence peptidique R9M. La présente divulgation décrit des peptides comprenant une séquence peptidique d'au moins neuf (9) acides aminés consécutifs dérivée substantiellement de la séquence peptidique R9M sauvage.

Par peptide immunogène on entend un peptide capable d'induire une réponse cellulaire ou humorale. Préférablement, les peptides immunogéniques de l'invention sont caractérisés en ce qu'ils ralentissent le développement de cellules tumorales lymphoblastiques et induisent une réponse immunitaire contre le développement de cellules tumorales lymphoblastiques après leur administration à un patient leucémique. Parmi les peptides préférés de la présente invention, on retrouve les peptides ayant comme séquence peptidique: RIAESILGM, RIAEAILGM, RIAE-α *but*ILGM, YIAESILGM, YIAEAILGM, et YIAE*α*-*but*ILGM.

L'invention porte également sur les polynucléotides codant pour les peptides immunogéniques R9M mutés, les vecteurs d'expression cellulaire comportant les séquences d'acides nucléiques exprimant les peptides immunogéniques R9M mutés et les anticorps polyclonaux ou monoclonaux capables de se fixer sur au moins un des peptides/polynucléotides mentionnés précédemment.

Dans un mode de réalisation privilégié de l'invention, ledit vecteur d'expression cellulaire comporte une séquence d'acide nucléique choisie dans le groupe constitué par les SEQ ID NOs: 19 à 38.

L'invention porte aussi sur des médicaments et des compositions pharmaceutiques comprenant les peptides, les polynucléotides et/ou les anticorps objets de l'invention.

La présente invention concerne également l'utilisation d'au moins un peptide immunogénique tel que décrit ci-dessus, et/ou d'au moins un polynucléotide tel que défini ci-dessus pour la préparation d'un agent antitumoral ou pour la préparation d'un vaccin antitumoral.

Un des avantages majeurs de la présente invention est que les peptides R9M mutés obtenus présentent une haute affinité pour la molécule HLA-A2.01 et forment avec cette molécule un complexe stable les rendant capables d'induire *in vivo* ou *in vitro* une réponse immunitaire cytotoxique.

De nombreux autres objectifs et avantages de la présente invention apparaîtront à la lecture de la description non-limitative de l'invention qui suit.

### BRÈVE DESCRIPTION DES DESSINS

La **Figure 1** montre le peptide épitopique R9M sauvage.
La **Figure 2** illustre à l'aide de graphiques la réponse primaire de cellules CTL humaines contre des cellules-cibles HLA-A2.1⁺T2 stimulées avec différents peptides. **Colonne A** = cellules T2 stimulées avec les peptides R9M mutés; **Colonne B** = cellules T2 stimulées avec le peptide R9M sauvage.
La **Figure 3** illustre à l'aide de graphiques la provocation de tumeurs chez des souris HHD par des cellules EL4 S3-Rob-HHD exprimant la protéine de fusion TEL/AM1.
La **Figure 4** illustre le taux de survie de souris HHD immunisées avec le peptide R9M sauvage ou les peptides R9M mutés, les souris ayant des tumeurs provoquées par des cellules EL4 S3-Rob-HHD exprimant la protéine de fusion TEL/AM1.

### DESCRIPTION DÉTAILLÉE DE L'INVENTION

Le peptide R9M sauvage a comme séquence peptidique Arg-Ile-Ala-Glu-Cys-Ile-Leu-Gly-Met (RIAECILGM; SEQ ID NO: 1). Cette séquence peptidique correspond à une région de la protéine humaine de fusion TEL/AML1 (GENBANK™ no S78496).

La présente invention se rapporte à l'optimisation du peptide R9M et à l'utilisation des peptides obtenus pour la vaccination thérapeutique et/ou la vaccination préventive de la leucémie chez l'homme. La nomenclature utilisée pour décrire la séquence des peptides de la présente invention est la nomenclature internationale utilisant le code à trois lettres ou le code à une lettre, et où l'extrémité amino-terminale est présentée à gauche et l'extrémité carboxy-terminale est présentée à droite.

Il convient de rappeler que dans l'ensemble de la description, on entend désigner par "acide aminé" aussi bien les acides aminés naturels que les acides aminés non--naturels. Par "acide aminé naturel", on entend désigner les acides aminés sous forme L que l'on peut trouver dans les protéines naturelles, c'est-à-dire alanine, arginine, asparagine, acide aspartique, cystéine, glutamine, acide glutamique, glycine, histidine, isoleucine, leucine, lysine, méthionine, phénylalanine, proline, sérine, thréonine, tryptophane, tyrosine et valine.

Cependant, la présente invention concerne également les peptides ayant des acides aminés non--naturels, c'est-à-dire les acides aminés précédents sous leur forme D, ainsi que les formes homo de certains acides aminés comme l'arginine, la lysine, la phénylalanine et la sérine ou les formes nor de la leucine ou de la valine. Dans certains cas, il est avantageux d'utiliser l'acide alpha-aminobutyrique, et il est également possible d'envisager l'utilisation d'autres acides aminés comme par exemple l'agmatine (Agm), l'acide alpha-aminoisobutyrique (Aib), le N-formyle-Trp (F-Trp), la sarcosine, la statine, l'ornithine, la désaminotyrosine et autres acides aminés modifiés. Tous les acides aminés peuvent être utilisés à condition qu'ils ne soient pas toxiques à l'humain et qu'ils ne modifient pas la capacité des peptides obtenus à induire une réponse immunitaire *in vivo* ou *in vitro.*

Par "peptide dérivé" d'une protéine sauvage, on entend tous les peptides qui possèdent une séquence peptidique substantiellement identique, au moins en partie, à la séquence peptidique de la protéine sauvage. Il peut s'agir par exemple de peptides modifiés chimiquement ayant une séquence peptidique 100% identique à une portion de la protéine sauvage. Il peut s'agir aussi de peptides hybrides ayant une première portion 100% identique à une première portion de la protéine sauvage et une seconde portion aucunement/partiellement identique à une seconde portion de la protéine sauvage. Il peut s'agir encore de peptides ayant une homologie totale/partielle avec une portion de la protéine sauvage. La présente divulgation décrit des peptides comprenant une séquence peptidique dérivée substantiellement de la séquence peptidique R9M de la protéine humaine de fusion TEL/AML1, cette séquence ayant au moins neuf (9) acides aminés consécutifs.

Par peptides "mutés" dérivés d'une protéine sauvage, on entend tous les peptides qui ont été obtenus suite à une modification de ladite protéine sauvage par substitution de un ou plusieurs des acides aminés de la protéine sauvage. Il peut également s'agir d'une modification apportée par l'addition de chaînes carbonées fixées sur au moins un des acides aminés des peptides pour lesquels il existe une substitution de l'un des acides aminés par rapport à la protéine sauvage. La présente invention couvre les peptides qui dérivent de la protéine humaine de fusion TEL/AML1 qui comprend l'épitope sauvage R9M et qui ont été mutés dans la séquence R9M. La présente invention couvre toutefois tout polypeptide comprenant une séquence peptidique mutée ou dérivée de la séquence sauvage R9M et capable de produire des effets biologiques au moins équivalents à ceux dont la séquence spécifique est fournie ci-après.

Une personne versée dans le domaine de l'invention saura obtenir différents peptides dérivés et mutés et elle saura également déterminer quels sont, parmi tous les peptides obtenus, les peptides ayant une activité biologique adéquate. Préférablement, la séquence du peptide (incluant ses modifications chimiques le cas échéant) sera telle que le peptide obtenu présentera une affinité accrue pour la molécule HLA-A2.01 de sorte qu'il puisse former avec cette molécule un complexe stable ayant une demi-vie supérieure (DC₅₀) à la demi-vie du complexe peptide-R9M sauvage/HLA-A2.01. Le terme DC₅₀ caractérisant la demi-vie du complexe peptide HLA-A2.01 est décrit dans la publication "H-2 class 1 knockout, HLA-A2.1-transgenic mice: a versatile animal model for preclinical évaluation of antitumor immunotherapeutic strategies" (European Journal of Immunology : 29:3112-21, 1999). Cette demi-vie devrait normalement rendre capable d'induire *in vivo* ou *in vitro* une réponse immunitaire cytotoxique. Préférablement, le peptide obtenu ralentira la croissance de cellules tumorales lymphoblastiques lorsque administré à un receveur leucémique et déclenchera une réponse T-cytotoxique contre les cellules leucémiques de type LAL-B commune avec t(12;21) du receveur leucémique.

Dans un mode de réalisation privilégié de l'invention, ledit complexe possède une demi-vie comprise entre 0,9 heure et environ 5 heures.

De manière avantageuse, les inventeurs ont découvert que la substitution de l'acide aminé cystéine en position 5 de la séquence peptidique R9M sauvage par un acide aminé neutre choisi parmi la sérine, l'alanine et l'acide alpha-aminobutyrique et/ou la substitution de l'acide aminé arginine en position 1 de la séquence peptidique R9M sauvage par un acide aminé aromatique telle que la tyrosine conférait aux peptides mutés une activité biologique accrue. À cet effet, les inventeurs ont découvert que les peptides ayant comme séquence peptidique RIAESILGM (SEQ ID NO: 2), RIAEAILGM (SEQ ID NO: 3), YIAESILGM (SEQ ID NO: 4), YIAEAILGM (SEQ ID NO: 5), RIAE*α*-*but*ILGM (SEQ ID NO: 6), et YIAE*α*-*but*ILGM (SEQ ID NO: 7), étaient particulièrement intéressants. Par "*α-but*", on entend un acide alpha-aminobutyrique qui est fixé sur l'acide glutamique.

Dans un mode de réalisation privilégié de l'invention, le peptide immunogénique consiste en une séquence peptidique choisie dans le groupe constitué par les SEQ ID NOs: 4, 5 et 7 ou comprend une séquence peptidique choisie dans le groupe constitué par les SEQ ID NOs: 4, 5 et 7.

Dans un autre mode de réalisation privilégié de l'invention, le peptide immunogénique consiste en une séquence peptidique définie par SEQ ID NO: 6 ou comprend une séquence peptidique définie par SEQ ID NO: 6.

Dans un autre mode de réalisation privilégié de l'invention, le peptide immunogénique consiste en une séquence peptidique de SEQ ID NO: 43 ou comprend une séquence peptidique de SEQ ID NO: 43.

Il est également possible de prévoir d'autres modifications (chimique ou peptidique) permettant aux peptides de franchir certaines barrières biologiques, de montrer une meilleure solubilisation, de faciliter leur incorporation dans des formes galéniques particulières comme par exemple des liposomes ou des microparticules. Il convient d'ailleurs de remarquer à ce propos que les peptides selon la présente divulgation peuvent se présenter sous forme déglycosylée ou glycosylée si cela est nécessaire.

Les peptides selon la présente invention peuvent être préparés par tout procédé approprié. Ils peuvent notamment être obtenus par synthèse chimique mais il est également possible de les obtenir par voie biologique en utilisant notamment différents vecteurs dans les cultures cellulaires appropriées tel que cela sera décrit ci-après. Il convient également de remarquer que, dans certains cas et suivant la méthode de préparation, il pourra être nécessaire de renaturer certaines structures tertiaires des peptides obtenus.

Les séquences d'ADN codant pour les peptides de l'invention peuvent être aisément déterminées à partir des séquences en acides aminés. Le Tableau 1 suivant donne la séquence nucléotidique du peptide R9M sauvage et les séquences nucléotidiques déduites de plusieurs peptides R9M mutés:

**TABLEAU 1:**

| **SÉQUENCES PEPTIDIQUES** | | **SÉQUENCE(S) NUCLÉOTIDIQUE(S)** | |
|---|---|---|---|
| **SÉQUENCE** | **SEQ ID NO:** | **SEQUENCE** | **SEQ ID NO:** |
| RIAECILGM (R9M sauvage) | 1 | AGA ATA GCA GAA **TGC** ATA CTT GGA ATG | 8 |
| RIAESILGM | 2 | AGA ATA GCA GAA **AGC** ATA CTT GGA ATG | 9 |
| | | AGA ATA GCA GAA **AGT** ATA CTT GGA ATG | 10 |
| | | AGA ATA GCA GAA **TCA** ATA CTT GGA ATG | 11 |
| | | AGA ATA GCA GAA **TCC** ATA CTT GGA ATG | 12 |
| | | AGA ATA GCA GAA **TCG** ATA CTT GGA ATG | 13 |
| | | AGA ATA GCA GAA **TCT** ATA CTT GGA ATG | 14 |
| RIAEAILGM | 3 | AGA ATA GCA GAA **GCA** ATA CTT GGA ATG | 15 |
| | | AGA ATA GCA GAA **GCC** ATA CTT GGA ATG | 16 |
| | | AGA ATA GCA GAA **GCG** ATA CTT GGA ATG | 17 |
| | | AGA ATA GCA GAA **GCT** ATA CTT GGA ATG | 18 |
| YIAESILGM | 4 | **TAC** ATA GCA GAA **AGC** ATA CTT GGA ATG | 19 |
| | | **TAT** ATA GCA GAA **AGC** ATA CTT GGA ATG | 20 |
| | | **TAC** ATA GCA GAA **AGT** ATA CTT GGA ATG | 21 |
| | | **TAT** ATA GCA GAA **AGT** ATA CTT GGA ATG | 22 |
| | | **TAC** ATA GCA GAA **TCA** ATA CTT GGA ATG | 23 |
| | | **TAT** ATA GCA GAA **TCA** ATA CTT GGA ATG | 24 |
| | | **TAC** ATA GCA GAA **TCC** ATA CTT GGA ATG | 25 |
| | | **TAT** ATA GCA GAA **TCC** ATA CTT GGA ATG | 26 |
| | | **TAC** ATA GCA GAA **TCG** ATA CTT GGA ATG | 27 |
| | | **TAT** ATA GCA GAA **TCG** ATA CTT GGA ATG | 28 |
| | | **TAC** ATA GCA GAA **TCT** ATA CTT GGA ATG | 29 |
| | | **TAT** ATA GCA GAA **TCT** ATA CTT GGA ATG | 30 |
| YIAEAILGM | 5 | **TAC** ATA GCA GAA **GCA** ATA CTT GGA ATG | 31 |
| | | **TAT** ATA GCA GAA **GCA** ATA CTT GGA ATG32 | 32 |
| | | **TAC** ATA GCA GAA **GCC** ATA CTT GGA ATG33 | 33 |
| | | **TAT** ATA GCA GAA **GCC** ATA CTT GGA ATG34 | 34 |
| | | **TAC** ATA GCA GAA **GCG** ATA CTT GGA ATG35 | 35 |
| | | **TAT** ATA GCA GAA **GCG** ATA CTT GGA ATG36 | 36 |
| | | **TAC** ATA GCA GAA **GCT** ATA CTT GGA ATG37 | 37 |
| | | **TAT** ATA GCA GAA **GCT** ATA CTT GGA ATG38 | 38 |

Dans un mode de réalisation privilégié de l'invention, le polynucléotide codant pour un peptide immunogénique selon l'invention a sa séquence nucléotidique choisie dans le groupe constitué par les SEQ ID NOs: 19 à 38.

Ainsi, l'invention a également pour objet un procédé de préparation d'un peptide de l'invention, par transformation d'un hôte cellulaire à l'aide d'un vecteur d'expression (plasmide, cosmide, virus, etc) comprenant les séquences d'ADN codant pour les peptides de l'invention, suivi de la mise en culture de l'hôte cellulaire ainsi transformé, et de la récupération du peptide dans le milieu de culture. La présente divulgation décrit tout hôte cellulaire transformé par un vecteur d'expression tel que défini ci-dessus, et comprenant les éléments de régulation permettant l'expression de la séquence de nucléotides codant pour un peptide selon l'invention. L'utilisation de vecteurs pour l'expression de protéines et de peptides dans les cellules d'un hôte, notamment l'humain, est connue et ne sera pas décrite en détail. Les constructions spécifiques dépendent évidemment de l'hôte, de l'épitope et du vecteur retenu.

Les peptides mutés de la présente invention et les polynucléotides les codant peuvent être utilisés de nombreuses façons comme agents antitumoraux ou pour la préparation d'un vaccin antitumoral. La présente invention vise un procédé de stimulation *in vitro* de la réponse immunitaire chez l'homme, comprenant la séparation des cellules lymphoïdes de l'homme et l'incubation *in vitro* desdites cellules en présence d'au moins un peptide immunogénique muté et/ou en présence d'au moins un polynucléotide objets de l'invention, lesdites cellules permettant d'induire une réponse cytotoxique *in vitro.* Ces cellules, qui expriment les épitopes issus de TEL/AML1, peuvent stimuler *in vitro* une réponse cytotoxique spécifique anti-leucémique, les cellules TCD8 stimulées *in vitro* étant ultérieurement injectées au patient leucémique. On peut aussi utiliser les peptides et polynucléotides objets de l'invention dans un procédé d'induction *in vivo* de la réponse TCD8 anti-leucémique, *via* l'injection de cellules exprimant les épitopes issus de TEL/AML1 décrites précédemment a un patient leucémique, les cellules injectées permettant l'induction d'une réponse TCD8 anti-leucémique *in vivo.*

L'invention vise l'utilisation de cellules cytotoxiques T CD8 issues de l'incubation *in vitro* de cellules lymphoïdes préparées *in vitro* selon ledit procédé de stimulation *in vitro,* pour la préparation d'un médicament destiné au traitement de la leucémie aiguë lymphoblastique LAL-B commune avec t(12;21).

L'invention vise également l'utilisation de cellules lymphoïdes préparées *in vitro* selon ledit procédé de stimulation *in vitro,* pour la préparation d'un médicament destiné au traitement de la leucémie aigüe lymphoblastique LAL-B commune avec t(12;21).

L'invention concerne un médicament susceptible de stimuler une réponse immunitaire *in vivo* chez l'homme, comprenant des cellules cytotoxiques T CD8 issues de l'incubation *in vitro* des cellules lymphoïdes stimulées *in vitro* selon ledit procédé de stimulation *in vitro,* ou des cellules lymphoïdes stimulées *in vitro* selon ledit procédé de stimulation *in vitro.*

L'invention vise également un anticorps polyclonal ou monoclonal, des cellules cytotoxiques T CD8 issues de l'incubation *in vitro* de cellules lymphoïdes préparées *in vitro* selon ledit procédé de stimulation *in vitro* ou des cellules lymphoïdes préparées *in vitro* selon ledit procédé de stimulation *in vitro,* pour utilisation dans le traitement de la leucémie aiguë lymphoblastique LAL-B commune avec t(12;21).

Les peptides mutés de la présente invention et les polynucléotides les codant peuvent aussi être utilisés pour préparer des anticorps polyclonal ou monoclonal se fixant sur au moins un peptide/polynucléotide objet de l'invention. La présente invention vise donc également de tels anticorps purifiés qui peuvent être obtenus par des techniques très bien connues.

Les peptides mutés de la présente invention et les polynucléotides les codant peuvent être utilisés pour la fabrication d'un médicament et ceci dans le but d'être administrés *in vivo à des* fins de vaccination thérapeutique et/ou de vaccination préventive de la leucémie chez l'homme, tout particulièrement la leucémie aiguë lymphoblastique LAL-B commune avec t(12;21). Ces médicaments peuvent comprendre au moins un des éléments choisi dans le groupe constitué par les peptides immunogéniques et les polynucléotides ci-dessus décrits et des cellules-T lytiques sensibilisées *in vitro* par la mise en contact d'un peptide immunogénique selon l'invention. Les anticorps polyclonaux ou monoclonaux mentionnés précédemment peuvent eux aussi être utilisés pour la préparation d'un médicament destiné au traitement de la leucémie aiguë lymphoblastique LAL-B commune avec t(12;21).

Dans un mode de réalisation privilégié de l'invention, au moins une portion des peptides immunogéniques selon l'invention est conjuguée à un support sur lequel elle est absorbée ou fixée de façon covalente ou noncovalente, à son extrémité C et/ou N-terminale. Le support peut être constitué de molécules-porteuses (naturelles ou synthétiques), physiologiquement acceptables et non toxiques. Ces molécules-porteuses peuvent permettre notamment d'augmenter l'immunogénicité des peptides de l'invention par l'intermédiaire de groupements réactifs complémentaires respectivement portés par la molécule-porteuse et le peptide. À titre d'exemple de molécules-porteuses, on mentionnera des protéines naturelles telles que l'anatoxine tétanique, l'ovalbumine, des sérums albumines, des hémocyamines, le PPD (purified protein derivative) de la tuberculine, etc. À titre de supports macromoléculaires synthétiques, on mentionnera par exemple des polylysines ou des poly(D-L-alanine)-poly(L-lysine). À titre de supports hydrocarbonés ou lipidiques, on mentionnera les acides gras saturés ou insaturés. Le support peut également prendre la forme de liposomes, de particules, de vésicules, de microsphères ou de billes de latex ou de polystyrène.

Dans un mode de réalisation privilégié de l'invention, le support est choisi dans le groupe constitué par les vésicules lipidiques, les microsphères, les billes de latex, les billes de polystyrène et les protéines.

L'invention porte aussi sur des compositions thérapeutiques comprenant un médicament ou plusieurs anticorps polyclonaux ou monoclonaux tels que décrits précédemment et un véhicule pharmaceutique acceptable. Ces compostions peuvent être avantageuses pour le traitement ou la prévention de la leucémie aiguë lymphoblastique B commune avec t(12;21) chez l'humain. Bien entendu, l'utilisation de compositions à base d'anticorps nécessite généralement que ceux ci soient compatibles avec l'administration à l'être humain. Il peut notamment s'agir d'anticorps humanisés par des techniques connues ou directement exprimés *in situ* à partir de la séquence d'ADN.

Les compositions thérapeutiques peuvent se présenter sous une forme solide ou liquide quelconque habituelle pour l'administration pharmaceutique, c'est-à-dire par exemple des formes d'administration liquide, en gel, ou tout autre support permettant par exemple la libération contrôlée. Parmi les compositions utilisables, on peut citer notamment les compositions injectables plus particulièrement destinées aux injections dans la circulation sanguine chez l'humain. Les compositions peuvent comporter également des composants augmentant l'immunogénicité des peptides, notamment d'autres peptides immunogènes, des adjuvants d'immunité spécifiques ou non tels que l'adjuvant de Freund, des polysaccharides ou des composés équivalents.

La présente divulgation décrit des compositions destinées à être administrées afin d'exprimer *in situ* les peptides décrits précédemment. Par exemple, en injectant l' "ADN nu" codant pour les peptides immunogéniques de l'invention, cette injection conduit, dans un certain nombre de cas, à l'expression du peptide codé et à une réponse immunitaire contre ledit peptide. On pourra également utiliser des systèmes d' "ADN nu" mais comportant leur propre système d'expression ou des vecteurs d'expression tel que décrit précédemment. Les vecteurs d'expression sont susceptibles, dans certains cas, d'améliorer leur activité des peptides exprimés. Les systèmes de vaccination mettant en oeuvre des séquences d'ADN sont connus et sont déjà largement décrits dans la littérature.

L'invention vise aussi les cellules tumorales obtenues par double transfection de souris EL4 S3-Rob (β2-microglobuline murine négative) avec:
1) le gène humain codant la molécule HHD; et
2) le gène humain codant pour la protéine de fusion TEL/AML1 comprenant :
   a) la séquence peptidique R9M sauvage (Arg-Ile-Ala-Glu-Cys-Ile-Leu-Gly-Met); ou
   b) une séquence peptidique R9M muté codant pour un peptide immunogénique muté tel que défini ci-dessus.

Ces cellules permettent l'obtention d'un modèle animal de vaccination anti-leucémique, ce qui valide les expériences de vaccination. Les méthodes pour obtenir ce genre de cellules sont bien connues dans le domaine et ne seront pas décrites en détail. La méthode pour obtenir les souris EL4 S3-Rob a été décrite en détails par Pascolo et coll. dans J. Exp Med., 185:2043-51 (1997).

La présente invention couvre tout particulièrement la lignée cellulaire doublement transfectée par les gènes codant pour la molécule HLA-A2.01 et la translocation TEL/AML1. Cette lignée cellulaire est appelée "*EL4 Rob.HHD.TEL.AML1*" et elle a fait l'objet d'un dépôt biologique auprès de la Collection Nationale de Culture de Microorganismes (CNCM, Institut Pasteur, Paris) le 1^{er} décembre 2000, et s'est vue attribuer le No. d'enregistrement I-2587.

L'invention vise encore un procédé de sélection de molécules thérapeutiques pouvant induire une réponse immunitaire protectrice *in vivo* contre des peptides sauvages tumoraux, viraux, bactériens, ou fongiques. Ce procédé est caractérisé en ce que:
1) on administre à un modèle animal:
   a) les molécules à tester (groupe 1) ou lesdits peptides sauvages tumoraux, viraux, bactériens ou fongiques
   b) une lignée cellulaire tumorale EL4S3-rob doublement transfectée par des séquences nucléotidiques codant pour la molécule HHD et des séquences nucléotidiques codant pour lesdits peptides sauvages tumoraux, viraux, bactériens ou fongiques,
   ledit modèle animal ayant un génotype compatible avec celui de ladite cellule tumorale transfectée;
2) on compare la réponse immunitaire induite dans chacun des groupes contre les épitopes desdits peptides tumoraux, viraux, bactériens, fongiques;
3) on sélectionne les molécules ayant induit une réponse immunitaire *in vivo* contre les épitopes desdits peptides tumoraux, viraux, bactériens, fongiques supérieure à celle induite par lesdits peptides sauvages tumoraux, viraux, bactériens, ou fongiques.

Le modèle animal compatible peut être obtenu en utilisant d'une part une souris ayant un CMH inactif et non exprimé remplacé par le gène codant pour HLA-A2.01 et, d'autre part, une lignée cellulaire tumorale transfectée par au moins le gène codant pour la molécule HLA-A2.01. Il est toutefois entendu que l'invention s'étend à toute construction de modèle animal compatible, par exemple en remplaçant le gène codant pour HLA-A2.01 par un gène codant pour HLA-B7 etc.

Bien qu'à travers le mémoire descriptif de la présente invention on utilise le terme "peptide", il est entendu que l'invention ne se limite pas aux composés formés par l'union d'un nombre limité d'acides aminés. En effet, la souplesse des technologies recombinantes permet de réaliser des protéines comportant une pluralité d'épitopes identiques ou différents et susceptibles d'améliorer l'activité immunogénique du produit final. Ainsi la présente invention couvre également les polymères immunogéniques comprenant entre deux et dix peptides choisis parmi les peptides mutés définis précédemment et les peptides consistant en ou comprenant SEQ ID NOs: 4 à 7. De même, la présente invention couvre les polynucléotides codant pour une séquence peptidique autre qu'une séquence R9M sauvage, lesdits polynucléotides incorporant:
- plusieurs polynucléotides codant pour des peptides immunogéniques tels que définis précédemment; et
- plusieurs polynucléotides dont la séquence est choisie dans le groupe constitué d'une séquence comprenant ou consistant en SEQ ID NO: 19 à 38.

Enfin, la présente invention inclut les oligonucléotides ayant une séquence nucléotidique codant pour une séquence peptidique autre qu'une séquence R9M sauvage, ces oligonucléotides incorporant un ou plusieurs polynucléotides tels que définis précédemment.

Les exemples ci-après permettront de mettre en évidence d'autres caractéristiques et avantages de la présente invention.

### EXEMPLES

Les exemples qui suivent servent à illustrer l'étendue d'utilisation de la présente invention et non à limiter sa portée. Des modifications et variations peuvent y être effectuées sans que l'on échappe à l'esprit et à la portée de l'invention. Bien que l'on puisse utiliser d'autres méthodes ou produits équivalents à ceux que l'on retrouve ci-dessous pour tester ou réaliser la présente invention, le matériel et les méthodes préférés sont décrits.

### Exemple 1 : Optimisation des peptides épitopiques de la région de fusion TEL/AML1

Il est connu que l'affinité d'un peptide épitopique pour le CMH prédit en partie son potentiel immunogénique. Cette affinité peut se mesurer par sa capacité de fixation au CMH de classe I et par la stabilité conférée au complexe CMH peptide ainsi formé à la surface cellulaire en utilisant des cellules T2 tap- exprimant HLA-A*02.01.

Les inventeurs de la présente invention ont observé que le complexe R9M/HLA A2.01 à la surface des cellules T2 était peu stable. Ces constatations ont été mises en relation avec l'instabilité physico-chimique du peptide due à la présence d'une cystéine en position 5 susceptible de favoriser une oxydation et la formation de composés secondaires *via* le groupement SH. Des articles récents montrent que la dimérisation et la cystéinisation de peptides synthétiques contenant la cystéine surviennent *in vitro* mais aussi *in vivo* et que ces processus modifient fortement l'immunogénicité de ces peptides. La présence d'agents réducteurs ou la substitution de la cystéine par l'acide α-aminobutyrique ou la sérine accroît l'immunogénicité de ces peptides.

Des efforts répétés d'immunisation des souris transgéniques HLA A2.01 avec le peptide R9M ont montré l'inconstance et la faiblesse des réponses de CTL induites. La cystéine du peptide R9M a donc été substituée par un acide aminé neutre comme la sérine ou l'alanine ou par l'acide α-aminobutyrique. Les résultats ci-dessus montrent que cette substitution accroît effectivement la stabilité du complexe peptide-HLA-A*02.01. La substitution de l'acide aminé en position 1 par une tyrosine permet aussi d'accroître l'immunogénicité des peptides HLA-A*02.01 restreints.

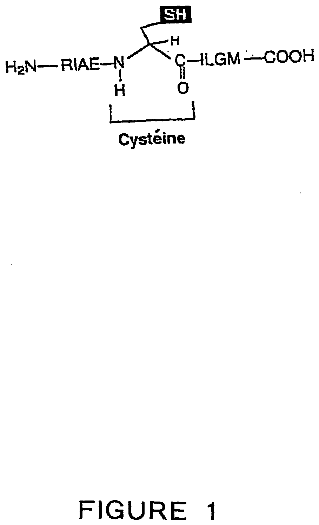

Ces résultats préliminaires ont, par la suite, été utilisés pour optimiser davantage le peptide R9M. Les résultats qui suivent montrent que la simple ou double substitution en P5 par un des acides aminés neutres (alanine, sérine ou acide α-aminobutyrique) et/ou en P1 par la tyrosine accroît les capacités antigéniques de ces peptides mutés (Figure 1). Les tests de fixation réalisés *in vitro* en utilisant les molécules HLA-A2.01 solubles et des cellules T2 ont montré que la capacité de fixation et de stabilisation des molécules HLA-A2.01 était plus importante comparée au peptide R9M sauvage (Tableau 2).

L'immunogénicité des peptides mutés a ensuite été étudiée chez la souris transgénique HHD (H2 classe 1-négative et transgénique pour HLA-A*02.01). Ces souris sont décrites dans European Journal of Immunology, 29:3112-21, 1999. Des groupes de plus de 10 souris HHD ont été immunisés par les peptides synthétiques (émulsion dans IFA avec le peptide auxiliaire HBVcore128-140 tel que décrit précédemment dans Firat et coll., Eur. J. Immunol. 29:3112-21 (1999)).

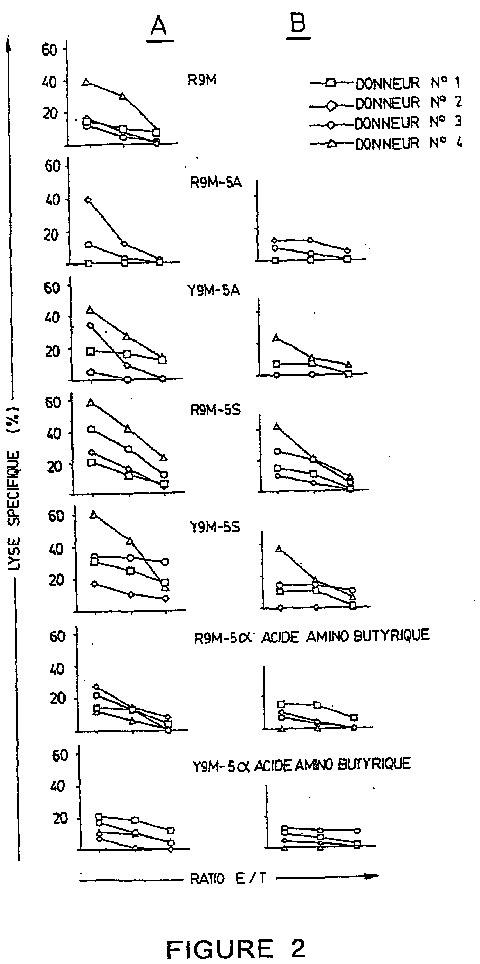

Les souris HHD immunisées par le peptide natif R9M développent une faible réponse CTL spécifique néanmoins capable de reconnaître la lignée tumorale TEL AML⁺ (Tableau 3 et Tableau 4). Par contre, les peptides mutés (ayant des substitutions simples ou doubles) induisent une activité CTL forte et une lyse des lignées tumorales TEL AML⁺ dans la majorité des cas (Tableau 3 et Tableau 4). L'immunogénicité accrue des peptides R9M mutés a ensuite été confirmée *in vitro* en utilisant des cellules mononucléées de 5 sujets sains HLA-A*02.01 (Figure 2).

**Tableau 2 : Liaison des peptides dérivés de la protéine de fusion native ETV6-AMLT et de leurs molécules analogues mutées, à la molécule HLA-A2.01.**

| | **Séquence en acide aminé** | **Mesure de la stabilisation** | | **Ratio peptide/¹²⁵ I-F10V** | |
|---|---|---|---|---|---|
| **Peptide** | | **RA^{b}** | **DC₅₀(h)**^{e} | **10/1** | **25/1** |
| **A9E** | AMPIGRIAE (SEQ ID NO: 39) | 10-100 | 2-4 | 47^{d} | 57 |
| **Y9E** | YMPIGRIAE (SEQ ID NO: 40) | >100 | 0 | 39 | 47 |
| **P9I** | PIGRIAECI (SEQ ID NO: 41) | >100 | 0 | 23 | 58 |
| **Y9I** | YIGRIAECI (SEQ ID NO: 42) | >100 | <2 | 43 | 60 |
| **R9M** | RIAECILGM (SEQ ID NO: 1) | 2,9 | <2 | 64 | 69 |
| **Y9M** | **Y**IAECILGM (SEQ ID NO: 43) | >100 | <2 | 45 | 66 |
| **R9M-5A** | RIAE**A**ILGM (SEQ ID NO: 3) | 3,2 | <2 | 59 | 72 |
| **Y9M-5A** | **Y**IAE**A**ILGM (SEQ ID NO: 5) | 0,9 | 2-5 | 79 | 87 |
| **R9M-5S** | RIAE**S**ILGM (SEQ ID NO: 2) | 2,2 | 2 | 74 | 75 |
| **Y9M-5S** | **Y**IAE**S**ILGM (SEQ ID NO: 4) | 1,4 | 2-5 | 25 | 37 |
| **R9M-5α-but^{a}** | RIAE***α*-*but***ILGM (SEQ ID NO: 6) | 2 | 2 | 27 | 49 |
| **Y9M-5α-but^{a}** | **Y**IAE***α*-*but***ILGM (SEQ ID NO: 7) | 0,7 | 2-5 | 26 | 26 |
| **HIV 1 rt.476** | ILKEPVHGV (SEQ ID NO: 44) | 1 | 5 | NT | NT |
| **F10V** | FLPSDYFPSV (SEQ ID NO: 45) | NT | NT | 63 | 84 |

| | | | | | |
|---|---|---|---|---|---|
| a) Substitution du résidu cystéine par l'acide α-amino butyrique. b) RA représente le ratio des concentrations nécessaires des échantillons testés versus le peptide en référence pour atteindre 20% du nombre maximal de molécules stabilisées tel que défini avec de fortes concentrations du peptide en référence. c) Demi-vie (DC₅₀) des complexes stabilisés peptide-HLA-A2.1 mesurée suivant une incubation pendant une nuit des cellules T2 et du peptide en référence. Obtenu en mesurant par immunofluorescence indirecte et une analyse FACS la surface cellulaire résiduelle des complexes peptide-HLA-A2.01 à différents intervalles (0, 2, 4 et 5 h). d) Représente le pourcentage d'inhibition de la liaison du peptide du virus de l'hépatite BHBVc.18-27 (F10V). | | | | | |

**Tableau 3 : Étude comparative de la réponse secondaire des cellules CTL provenant de souris HHD immunisées avec le peptide R9M sauvage ou les peptides R9M mutés.**

| **Effecteurs** | **Cellules RMAS-HHD stimulées avec le peptide muté** | ^{a}**Cellules RMAS-HHD stimulées avec le peptide R9M sauvage** |
|---|---|---|
| | ^{b}R/T (% de lyse nette) | ^{b}R/T (% de lyse nette) |
| **R9M** | 1/18 (13) | - |
| **R9M-5A** | 2/10 (40,43) | 2/10 (22,28) |
| **Y9M-5A** | 8/10 (27,44,51,62,65,65,66,77) | 7/10 (11,14,18,19,34,46,50) |
| **R9M-5S** | 11/14 (15,20,24,29,37,37,39,43,64,66,72) | 6/14 (11,11,17,50) |
| **Y9M-5S** | 9/10 (26,35,49,52,53,57,64,65,68) | 3/10 (14,43,45) |
| **R9M-5α-but** | 7/10 (14,15,30,43,52,71,75) | 5/10 (18,32,44,58,64) |
| **Y9M-5α-but** | 8/10 (15,45,57,58,60,62,65,67) | 8/10 (21,29,38,46,54,54,56) |

| | | |
|---|---|---|
| a) Des cellules de la rate de souris co-injectées s.c. avec le peptide sauvage RM9 et ses analogues mutés et le peptide auxiliaire HBVc.128 dans IFA onze (11) jours auparavant, furent restimulées *in vitro* avec les différents peptides. Six (6) jours plus tard celles-ci furent évaluées à différents ratios E/T contre des cellules-cibles RMA-S-HHD possédant les différents peptides en référence ou un peptide-contrôle (Inf.m.58). b) R/T représente les souris-répondeurs versus le total des souris testées. Les souris furent considérées comme ayant répondu lorsqu'au moins 10% de lyse spécifique fut observée. Les valeurs entre parenthèses correspondent à la lyse maximale observée pour chaque souris-répondeur à un ratio E/T 60:1. | | |

**Tableau 4 : Étude comparative de la réponse tertiaire de cellules CTL de souris HHD immunisées avec le peptide R9M sauvage ou les peptides R9M mutés.**

| **Effecteurs** | **Cellules RMAS-HHD stimulées avec le peptide muté** | **^{a}Cellules TEL/AML⁺ EL4-HHD** |
|---|---|---|
| | ^{b}R/T (% de lyse nette) | ^{b}R/T (% de lyse nette) |
| **R9M** | 2/10 (13,25) | 5/10 (15,15,22,30,33) |
| **R9M-5A** | 1/10 (20) | 6/10 (17,27,32,38,43,58) |
| **Y9M-5A** | 9/10 (10,40,44,51,62,65,65,66,77) | 5/10 (15,24,24,32,34) |
| **R9M-5S** | 6/10 (20,29,39,43,64,72) | 11/14 (12,14,15,20,24,29,31,32,34,36,56) |
| **Y9M-5S** | 9/10 (16,26,35,52,53,57,64,65,68) | 6/10 (13,17,22,25,32,60) |
| **R9M-5α-but** | 8/10 (12,25,14,15,30,43,71,75) | 7/10 (11,15,25,26,31,33,43) |
| **Y9M-5α-but** | 8/10 (18,45,65,65,68,69,69,75) | 10/10 (11,22,28,28,31,36,39,59,69) |

| | | |
|---|---|---|
| a) Des cellules de la rate de souris co-injectées s.c. avec le peptide sauvage RM9 et ses analogues mutés et le peptide auxiliaire HBVc.128 dans IFA onze (11) jours auparavant, furent restimulées à deux (2) reprises *in vitro* avec des trophoblastes-LPS possédant les peptides en référence. Les cellules furent évaluées six (6) jours plus tard à différents ratios E/T contre des cellules-cibles RMA-S-HHD possédant les différents peptides en référence ou un peptide-contrôle (Inf.m.58) ou contre des cellules EL4-HHD exprimant ou non le gène *tel*/*am*/*.* b) R/T représente les souris-répondeurs versus le total des souris testées. Les souris furent considérées comme ayant répondu lorsqu'au moins 10% de lyse spécifique fut observée. Les valeurs entre parenthèses correspondent à la lyse maximale observée pour chaque souris-répondeur à un ratio E/T 60:1. | | |

### Exemple 2: Élaboration d'un modèle tumoral chez la souris HHD

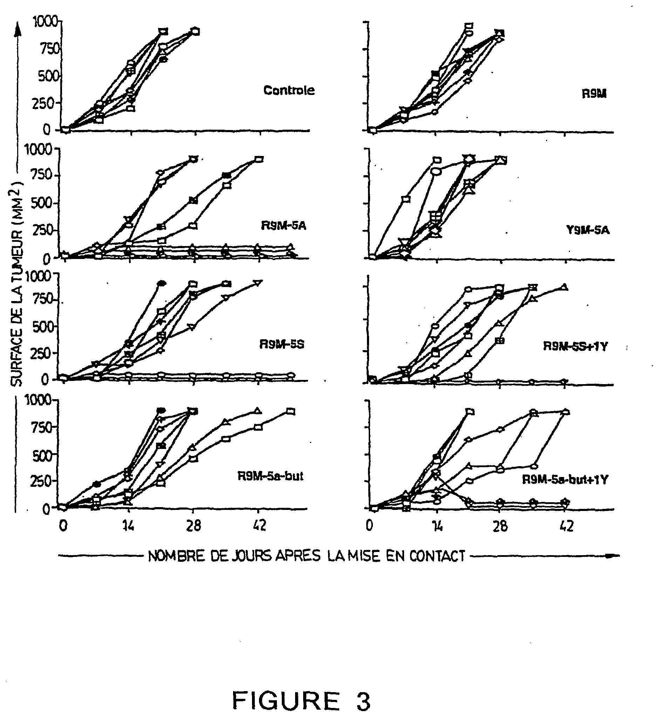

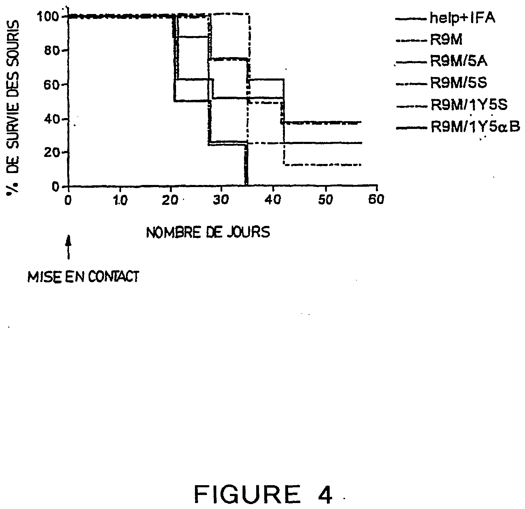

L'induction d'une réponse CTL forte *in vivo* ne reflète pas toujours la capacité protectrice de cette réponse immune contre les cellules tumorales. Ainsi, la lignée tumorale EL4 S3-Rob (β2-microglobuline murine négative) dérivée de souris C57BI/6 et de même fond génétique que les souris HHD, a été doublement transfectée par la molécule HHD et le gène de TEL/AML1. Après passages successifs, d'abord chez des souris *nude,* puis chez des souris HHD, les inventeurs ont obtenu une lignée fortement tumorigène chez la souris HHD. L'injection de ces cellules tumorales chez des groupes de 8 souris HHD préalablement immunisées avec le peptide R9M sauvage ou des peptides R9M mutés a montré que seuls ces derniers, en particulier R9M-5A, R9M-5α-butyrique et R9M-5S étaient capables de ralentir le développement de tumeurs (Figure 3) et de prolonger la survie des souris (Figure 4). Chez certaines souris, les inventeurs ont observé une protection totale.

### Conclusion

L'identification de peptides épitopiques uniquement exprimés par des cellules leucémiques t(12;21) rend envisageable une approche immuno-thérapeutique chez des patients. La présente invention rend disponible des méthodes et du matériel biologiques permettant l'évaluation de préparations vaccinales (modèle animal humanisé pour la réponse cytotoxique CD8 restreinte, modèle tumoral, série de peptides optimisés).

Différentes stratégies d'immunisation de souris HHD utilisant l'épitope R9M natif et ses analogues mutés permettront d'optimiser encore plus la présente invention afin d'induire une réponse forte et durable et conférer une protection efficace sans effets secondaires. En utilisant une stratégie biphasique (phase exploratoire murine, phase de validation humaine), il sera possible de présélectionner rapidement les formulations vaccinales proposables en clinique. Les meilleurs peptides R9M mutés pourront être testés à la fois seuls et en association dans différents vecteurs vaccinaux.

L'approche d'immunothérapie passive consistant à réinjecter aux patients des CTL induits *in vitro* pourra aussi être évaluée chez des souris HHD porteuses de tumeur EL4-S3-Rob-HHD exprimant la protéine de fusion TEL-AML1.

L'efficacité des cellules dendritiques chargées par des peptides ou transduites de manière stable pourrait aussi être étudiée puisque ces cellules sont actuellement utilisées dans différentes pathologies tumorales humaines et semblent être efficaces dans certains cas.

Le potentiel immunogénique des polynucléotides codant pour les peptides R9M mutés pourra aussi être évalué, que ce soit par des injections d'ADN nu ou par l'utilisation de vecteurs. En effet, il est connu que les vecteurs chimiques ou viraux permettent, par un meilleur ciblage cellulaire et une prolongation de la présentation antigénique, d'augmenter l'intensité de la réponse immunitaire. Plusieurs vecteurs pourraient donc être utilisés en tant que vecteurs vaccinaux. Parmi les vecteurs que l'on pourrait utiliser, il y ceux récemment développés à l'Institut Pasteur, soient:
i) le vecteur pCMV-B10 codant des glycoprotéines recombinantes du virus de l'Hépatite B dont les effets immunothérapeutiques dans le traitement d'affections cancéreuses ont déjà été établis;
ii) le vecteur Rougeole recombinant générateur de réponses immunitaires fortes chez les individus non déjà immuns contre la rougeole;
iii) les vecteurs lentiviraux à triplex qui induisent chez la souris des réponses CTL initiales et mémoire très efficaces et qui sont des plus performants pour induire des réponses humaines *in vitro* après stimulation par des cellules dendritiques;
iv) le vecteur ALVAC™ recombinant développé par Aventis-Pasteur; et
v) les lipopeptides qui sont actuellement utilisés en France dans plusieurs essais cliniques.

Bien que la présente invention ait été décrite par rapport aux réalisations concrètes et privilégiées, il apparaîtra toutefois évident aux personnes versées dans l'art ou la science en cause qu'il est possible d'introduire un certain nombre de variations et modifications sans déroger à la portée de l'invention décrite dans ce document.

## Revendications

1. Peptide immunogénique **caractérisé en ce qu'**il dérive de la protéine humaine de fusion TEL/AML1 comprenant la séquence peptidique R9M sauvage (SEQ ID NO: 1) et **en ce qu'**il est muté dans ladite séquence peptidique R9M par substitution de l'acide aminé 1 par un acide aminé aromatique, notamment par la tyrosine.

2. Peptide immunogénique, **caractérisé en ce qu'**il dérive de la protéine humaine de fusion TEL/AML1 comprenant la séquence peptidique R9M sauvage (SEQ ID NO: 1), et **en ce qu'**il est muté dans ladite séquence peptidique R9M par substitution de l'acide aminé 5 par un acide alpha-aminobutyrique.

3. Peptide immunogénique **caractérisé en ce qu'**il est obtenu du peptide immunogénique de la revendication 1, par substitution de l'acide aminé 5 de ladite séquence peptidique R9M par un acide aminé neutre choisi parmi la sérine, l'alanine, et l'acide alpha-aminobutyrique.

4. Peptide immunogénique selon la revendication 1 **caractérisé en ce qu'**il consiste en ladite séquence peptidique R9M substituée dans son acide aminé 1 par un acide aminé aromatique, notamment par la tyrosine.

5. Peptide immunogénique selon la revendication 3, **caractérisé en ce qu'**il consiste en ladite séquence peptidique R9M substituée dans son acide aminé 1 par un acide aminé aromatique, notamment par la tyrosine et substituée dans son acide aminé 5 par un acide aminé neutre choisi parmi la sérine, l'alanine, et l'acide alpha-aminobutyrique.

6. Peptide immunogénique selon la revendication 2, **caractérisé en ce qu'**il consiste en ladite séquence peptidique R9M substituée dans son acide aminé 5 par un acide alpha-aminobutyrique.

7. Peptide immunogénique selon la revendication 3 ou 5, **caractérisé en ce qu'**il consiste en une séquence peptidique choisie dans le groupe constitué par les SEQ ID NOs: 4, 5 et 7 ou **en ce qu'**il comprend une séquence peptidique choisie dans le groupe constitué par les SEQ ID NOs: 4, 5 et 7.

8. Peptide immunogénique selon la revendication 2 ou 6, **caractérisé en ce qu'**il consiste en une séquence peptidique définie par SEQ ID NO: 6 ou **en ce qu'**il comprend une séquence peptidique définie par SEQ ID NO: 6.

9. Peptide immunogénique selon la revendication 1 ou 4, **caractérisé en ce qu'**il consiste en une séquence peptidique de SEQ ID NO: 43 ou **en ce qu'**il comprend une séquence peptidique de SEQ ID NO: 43.

10. Peptide immunogénique selon l'une quelconque des revendications 1 à 9, **caractérisé en ce qu'**il ralentit la croissance de cellules tumorales lymphoblastiques après administration à un patient leucémique.

11. Peptide immunogénique selon l'une quelconque des revendications 1 à 10, **caractérisé en ce qu'**il induit une réponse immunitaire contre le développement de cellules tumorales lymphoblastiques après administration à un receveur.

12. Peptide immunogénique selon l'une quelconque des revendications 1 à 11, **caractérisé en ce que**, après son administration à un patient leucémique, il déclenche une réponse immunitaire *in vivo* contre les cellules leucémiques de type LAL-B commune avec t(12;21) dudit patient.

13. Peptide immunogénique selon l'une quelconque des revendications 1 à 11, **caractérisé en ce qu'**il déclenche une réponse immunitaire *in vitro* contre les cellules leucémiques de type LAL-B commune avec t(12;21) isolées d'un patient leucémique.

14. Peptide immunogénique selon l'une quelconque des revendications 1 à 11, **caractérisé en ce qu'**il déclenche une réponse cytotoxique *in vitro* contre les cellules leucémiques de type LAL-B commune avec t(12;21) isolées d'un patient leucémique.

15. Peptide immunogénique selon l'une quelconque des revendications 1 à 14, **caractérisé en ce qu'**il forme, du fait de sa haute affinité pour la molécule HLA-A2.01, un complexe stable avec cette molécule, le rendant capable d'induire *in vivo* ou *in vitro* une réponse immunitaire cytotoxique.

16. Peptide immunogénique selon la revendication 15, **caractérisé en ce que** ledit complexe possède une demi-vie comprise entre environ 0,9 heure et environ 5 heures.

17. Polynucléotide codant pour un peptide immunogénique selon l'une quelconque des revendications 1 à 16.

18. Polynucléotide de la revendication 17, **caractérisé en ce que** sa séquence nucléotidique est choisie dans le groupe constitué par les SEQ ID NOs: 19 à 38.

19. Médicament comprenant au moins un des éléments choisi dans le groupe constitué par :
a. le peptide immunogénique selon l'une quelconque des revendications 1 à 16;
b. le polynucléotide de la revendication 17 ou 18; et
c. des cellules T lytiques sensibilisées *in vitro* par la mise en contact d'un peptide immunogénique selon l'une quelconque des revendications 1 à 16.

20. Médicament selon la revendication 19, **caractérisé en ce que** au moins une portion des peptides immunogéniques comprise dans ledit médicament est conjuguée à un support sur lequel lesdits peptides sont absorbés ou fixés de façon covalente ou non-covalente.

21. Médicament selon la revendication 20, **caractérisé en ce que** le support est choisi dans le groupe constitué par les vésicules lipidiques, les microsphères, les billes de latex, les billes de polystyrène et les protéines.

22. Médicament selon l'une quelconque des revendications 19 à 21, pour la vaccination thérapeutique et/ou la vaccination préventive de la leucémie chez l'homme.

23. Médicament selon la revendication 22, **caractérisé en ce que** la leucémie est la leucémie aiguë lymphoblastique LAL-B commune avec t(12;21).

24. Composition thérapeutique comprenant un médicament selon l'une quelconque des revendications 19 à 23, et un véhicule pharmaceutique acceptable.

25. Composition thérapeutique selon la revendication 24, pour le traitement ou la prévention de la leucémie aiguë lymphoblastique B commune avec t(12;21) chez l'humain.

26. Utilisation d'au moins un peptide immunogénique sélectionné parmi les peptides définis dans les revendications 1 à 16 et/ou d'au moins un polynucléotide tel que défini à la revendication 17 ou 18, pour la préparation d'un agent antitumoral ou pour la préparation d'un vaccin antitumoral.

27. Procédé de stimulation *in vitro* de la réponse immunitaire chez l'homme, comprenant la séparation des cellules lymphoïdes de l'homme et l'incubation *in vitro* desdites cellules en présence d'au moins un peptide immunogénique sélectionné parmi les peptides définis dans les revendications 1 à 16 et/ou en présence d'au moins un polynucléotide selon la revendication 17 ou 18, lesdites cellules permettant d'induire une réponse cytotoxique *in vitro.*

28. Médicament susceptible de stimuler une réponse immunitaire *in vivo* chez l'homme, comprenant des cellules cytotoxiques T CD8 issues de l'incubation *in vitro* des cellules lymphoïdes stimulées *in vitro* selon le procédé de la revendication 27, ou des cellules lymphoïdes stimulées *in vitro* selon le procédé de la revendication 27.

29. Anticorps purifié polyclonal ou monoclonal, **caractérisé en ce qu'**il se fixe sur au moins un peptide selon l'une quelconque des revendications 1 à 16.

30. Utilisation d'un anticorps polyclonal ou monoclonal selon la revendication 29 pour la préparation d'un médicament destiné au traitement de la leucémie aiguë lymphoblastique LAL-B commune avec t(12;21).

31. Composition pharmaceutique comportant à titre de substance active un ou plusieurs anticorps polyclonaux ou monoclonaux tels que définis à la revendication 29 ou 30 avec un véhicule pharmaceutique acceptable.

32. Vecteur d'expression cellulaire, **caractérisé en ce qu'**il comporte une séquence d'acide nucléique exprimant un peptide immunogénique tel que défini selon l'une quelconque des revendications 1 à 16.

33. Vecteur de la revendication 32, **caractérisé en ce que** ladite séquence d'acide nucléique est choisie dans le groupe constitué par les SEQ ID NOs: 19 à 38.

34. Procédé de préparation d'un peptide immunogénique tel que défini selon l'une quelconque des revendications 1 à 16, comprenant la transformation d'un hôte cellulaire à l'aide d'un vecteur d'expression cellulaire tel que défini à la revendication 32 ou 33, suivi de la mise en culture de l'hôte cellulaire ainsi transformé, et de la récupération du peptide dans le milieu de culture.

35. Cellule tumorale **caractérisée en ce qu'**elle est obtenue par double transfection de souris EL4 S3-Rob (β2-microglobuline murine négative) avec:
1) le gène humain codant la molécule HHD; et
2) le gène humain codant pour la protéine de fusion TEL/AML1 comprenant la séquence peptidique R9M sauvage (SEQ ID NO: 1); ou une séquence peptidique R9M mutée codant pour un peptide immunogénique muté tel que défini selon l'une quelconque des revendications 1 à 16.

36. Cellule tumorale selon la revendication 35, déposée à la Collection Nationale de Culture de Microorganismes de l'Institut Pasteur (CNCM) le 1^{er} décembre 2000 sous le No. d'enregistrement I-2587.

37. Procédé de sélection de molécules thérapeutiques pouvant induire une réponse immunitaire protectrice *in vivo* contre des peptides sauvages tumoraux, viraux, bactériens ou fongiques, **caractérisé en ce que**:
1) on administre à un modèle animal:
a) les molécules à tester (groupe 1) ou lesdits peptides sauvages tumoraux, viraux, bactériens ou fongiques
b) une lignée cellulaire tumorale EL4S3-rob doublement transfectée par des séquences nucléotidiques codant pour la molécule HHD et des séquences nucléotidiques codant pour lesdits peptides sauvages tumoraux, viraux, bactériens ou fongiques,
ledit modèle animal ayant un génotype compatible avec celui de ladite cellule tumorale transfectée;
2) on compare la réponse immunitaire induite dans chacun des groupes contre les épitopes desdits peptides tumoraux, viraux, bactériens, fongiques;
3) on sélectionne les molécules ayant induit une réponse immunitaire *in vivo* contre les épitopes desdits peptides tumoraux, viraux, bactériens, fongiques supérieure à celle induite par lesdits peptides sauvages tumoraux, viraux, bactériens, ou fongiques.

38. Polymère immunogénique comprenant entre deux et dix peptides choisis parmi les peptides immunogéniques tels que définis aux revendications 1 à 16 et les peptides immunogéniques consistant en ou comprenant SEQ ID NOs: 4 à 7.

39. Polynucléotide codant pour une séquence peptidique autre qu'une séquence R9M sauvage, ledit polynucléotide incorporant :
- plusieurs polynucléotides codant pour des peptides immunogéniques tels que définis aux revendications 1 à 16 ; et
- plusieurs polynucléotides dont la séquence est choisie dans le groupe constitué d'une séquence comprenant ou consistant en SEQ ID NO:19 à 38.

40. Oligonucléotide ayant une séquence nucléotidique codant pour une séquence peptidique autre qu'une séquence R9M sauvage, ledit oligonucléotide incorporant un ou plusieurs polynucléotides selon la revendication 17 ou 18.

41. Utilisation de cellules cytotoxiques T CD8 issues de l'incubation *in vitro* de cellules lymphoïdes préparées *in vitro* selon le procédé de la revendication 27, pour la préparation d'un médicament destiné au traitement de la leucémie aiguë lymphoblastique LAL-B commune avec t(12;21).

42. Utilisation de cellules lymphoïdes préparées *in vitro* selon le procédé de la revendication 27, pour la préparation d'un médicament destiné au traitement de la leucémie aiguë lymphoblastique LAL-B commune avec t(12;21).

43. Peptide immunogénique muté tel que défini dans les revendications 1 à 16, pour utilisation dans l'induction *in vivo* d'une réponse cytotoxique T CD8⁺ anti-leucémique.

44. Utilisation d'un peptide immunogénique muté tel que défini dans les revendications 1 à 16, pour l'induction *in vitro* d'une réponse cytotoxique T CD8⁺.

45. Peptide immunogénique tel que défini dans les revendications 1 à 16 ou polynucléotide tel que défini à la revendication 17 ou 18, pour utilisation comme agent antitumoral ou vaccin antitumoral.

46. Anticorps polyclonal ou monoclonal selon la revendication 29, cellules cytotoxiques T CD8 issues de l'incubation *in vitro* de cellules lymphoïdes préparées *in vitro* selon le procédé de la revendication 27 ou cellules lymphoïdes préparées *in vitro* selon le procédé de la revendication 27, pour utilisation dans le traitement de la leucémie aiguë lymphoblastique LAL-B commune avec t(12;21).

## Patentansprüche

1. Immunogenes Peptid, **dadurch gekennzeichnet, dass** es abgeleitet ist vom humanen Fusionsprotein TEL/AML1, welches die Wildtyp-Peptidsequenz R9M (SEQ ID NO: 1) enthält, und dadurch, dass es in besagter Peptidsequenz R9M durch Substitution der Aminosäure 1 durch eine aromatische Aminosäure, insbesondere durch Tyrosin, mutiert ist.

2. Immunogenes Peptid, **dadurch gekennzeichnet, dass** es abgeleitet ist vom humanen Fusionsprotein TEL/AML1, welches die Wildtyp-Peptidsequenz R9M (SEQ ID NO: 1) enthält, und dadurch, dass es in besagter Peptidsequenz R9M durch Substitution der Aminosäure 5 durch α-Aminobuttersäure mutiert ist.

3. Immunogenes Peptid, **dadurch gekennzeichnet, dass** es erhalten wird aus dem immunogenen Peptid von Anspruch 1 durch Substitution der Aminosäure 5 besagter Peptidsequenz R9M durch eine neutrale Aminosäure, die Serin, Alanin oder α-Aminobuttersäure sein kann.

4. Immunogenes Peptid nach Anspruch 1, **dadurch gekennzeichnet, dass** es aus besagter Peptidsequenz R9M besteht, in welcher die Aminosäure 1 durch eine aromatische Aminosäure, insbesondere durch Tyrosin, ersetzt wird.

5. Immunogenes Peptid nach Anspruch 3, **dadurch gekennzeichnet, dass** es aus besagter Peptidsequenz R9M besteht, deren Aminosäure 1 durch eine aromatische Aminosäure, insbesondere durch Tyrosin, ersetzt wird, und deren Aminosäure 5 durch eine neutrale Aminosäure ersetzt wird, die Serin, Alanin oder α-Aminobuttersäure sein kann.

6. Immunogenes Peptid nach Anspruch 2, **dadurch gekennzeichnet, dass** es aus besagter Peptidsequenz R9M besteht, in welcher die Aminosäure 5 durch eine α-Aminobuttersäure ersetzt wird.

7. Immunogenes Peptid nach Anspruch 3 oder 5, **dadurch gekennzeichnet, dass** es aus einer Peptidsequenz besteht, die aus der Gruppe bestehend aus SEQ ID NOs: 4, 5 und 7 ausgewählt wird, oder dadurch, dass es eine Peptidsequenz enthält, die aus der Gruppe bestehend aus SEQ ID NOs: 4, 5 und 7 ausgewählt wird.

8. Immunogenes Peptid nach Anspruch 2 oder 6, **dadurch gekennzeichnet, dass** es aus einer Peptidsequenz besteht, die durch SEQ ID NO: 6 definiert wird oder dadurch, dass es eine Peptidsequenz enthält, die durch SEQ ID NO: 6 definiert wird.

9. Immunogenes Peptid nach Anspruch 1 oder 4, **dadurch gekennzeichnet, dass** es aus einer Peptidsequenz von SEQ ID NO: 43 besteht, oder dadurch, dass es eine Peptidsequenz SEQ ID NO: 43 enthält.

10. Immunogenes Peptid nach einem der Ansprüche 1 bis 9, **dadurch gekennzeichnet, dass** es das Wachstum lymphoblastischer Tumorzellen nach Verabreichung an einen leukämiekranken Patienten verlangsamt.

11. Immunogenes Peptid nach einem der Ansprüche 1 bis 10, **dadurch gekennzeichnet, dass** es eine Immunantwort gegen die Entwicklung lymphoblastischer Tumorzellen nach Verabreichung an einen Empfänger hervorruft.

12. Immunogenes Peptid nach einem der Ansprüche 1 bis 11, **dadurch gekennzeichnet, dass** es nach Verabreichung an einen leukämiekranken Patienten bei diesem *in vivo* eine Immunantwort gegen Leukämiezellen des Typs common-B-ALL mit t(12;21) auslöst.

13. Immunogenes Peptid nach einem der Ansprüche 1 bis 11, **dadurch gekennzeichnet, dass** es *in vitro* eine Immunantwort gegen die isolierten Leukämiezellen vom Typ common-B-ALL mit t(12; 21) eines leukämiekranken Patienten auslöst.

14. Immunogenes Peptid nach einem der Ansprüche 1 bis 11, **dadurch gekennzeichnet, dass** es *in vitro* eine zytotoxische Antwort gegen isolierte Leukämiezellen des Typs common-B-ALL mit t(12; 21) auslöst.

15. Immunogenes Peptid nach einem der Ansprüche 1 bis 14, **dadurch gekennzeichnet, dass** es aufgrund seiner hohen Affinität für das Molekül HLA-A2.01 einen stabilen Komplex mit diesem Molekül bildet, durch den es in der Lage ist, *in vivo* oder *in vitro* eine zytotoxische Immunantwort hervorzurufen.

16. Immunogenes Peptid nach Anspruch 15, **dadurch gekennzeichnet, dass** besagter Komplex eine Halbwertzeit zwischen ungefähr 0,9 Stunden und ungefähr 5 Stunden besitzt.

17. Polynucleotid, das für ein immunogenes Peptid nach einem der Ansprüche 1 bis 16 codiert.

18. Polynucleotid von Anspruch 17, **dadurch gekennzeichnet, dass** seine Nukleotidsequenz aus der Gruppe bestehend aus den SEQ ID NOs: 19 bis 38 ausgewählt wird.

19. Arzneimittel, welches mindestens eines der Elemente enthält, die aus der Gruppe ausgewählt werden, die gebildet wird:
a. durch das immunogene Peptid nach einem der Ansprüche 1 bis 16;
b. durch das Polynucleotid von Anspruch 17 oder 18; und
c. durch T-Zellen, die *in vitro* durch den Kontakt mit einem immunogenen Peptid nach einem der Ansprüche 1 bis 16 sensibilisiert wurden.

20. Arzneimittel nach Anspruch 19, **dadurch gekennzeichnet, dass** mindestens ein Abschnitt der im besagtem Arzneimittel enthaltenen immunogenen Peptide mit einer Trägersubstanz konjugiert ist, auf der die besagten Peptide kovalent oder nicht-kovalent absorbiert oder fixiert werden.

21. Arzneimittel nach Anspruch 20, **dadurch gekennzeichnet, dass** die Trägersubstanz ausgewählt wird aus der Gruppe bestehend aus Lipidvesikeln, sphärischen Mikropartikeln, Latexpartikeln, Polystyrolpartikeln und Proteinen.

22. Arzneimittel nach einem der Ansprüche 19 bis 21 für die therapeutische und/oder die vorbeugende Impfung gegen Leukämie beim Menschen.

23. Arzneimittel nach Anspruch 22, **dadurch gekennzeichnet, dass** es sich bei der Leukämie um eine akute lymphoblastische Leukämie des Typs common-B-ALL mit t(12;21) handelt.

24. Therapeutische Zusammensetzung bestehend aus einem Arzneimittel nach einem der Ansprüche 19 bis 23, und einem pharmazeutisch unbedenklichen Träger.

25. Therapeutische Zusammensetzung nach Anspruch 24 für die Behandlung oder Prävention von akuter lymphoblastischer Leukämie des Typs common-B-ALL mit t(12;21) beim Menschen.

26. Benutzung mindestens eines immunogenen Peptids, das aus den in den Ansprüchen 1 bis 16 definierten Peptiden ausgewählt wird, und/oder mindestens eines Polynucleotids gemäß der Definition von Anspruch 17 oder 18, das für die Herstellung eines antitumoralen Wirkstoffs oder für die Herstellung eines antitumoralen Impfstoffs ausgewählt wird.

27. *In vitro*-Stimulationsverfahren für eine Immunantwort beim Menschen, bestehend aus der Trennung der lymphoiden Zellen des Menschen und der *in vitro-*Inkubation besagter Zellen in Gegenwart mindestens eines immunogenen Peptids, das aus den in den Ansprüchen 1 bis 16 definierten Peptiden ausgewählt wird, und/oder in Gegenwart von mindestens einem Polynucleotid nach Anspruch 17 oder 18, wobei besagte Zellen *in vitro* das Hervorrufen einer zytotoxischen Antwort ermöglichen.

28. Arzneimittel, das beim Menschen eine Immunantwort *in vivo* stimulieren kann, welches zytotoxische Zellen T CD8 enthält, die aus der *in vitro*-Inkubation lymphoider Zellen stammt, die *in vitro* nach dem Verfahren von Anspruch 27 stimuliert wurden, oder lymphoider Zellen, die *in vitro* nach dem Verfahren von Anspruch 27 stimuliert werden.

29. Benutzung eines polyklonalen oder monoklonalen gereinigten Antikörpers, **dadurch gekennzeichnet, dass** er sich auf mindestens einem Peptid nach einem der Ansprüche 1 bis 16 fixiert.

30. Benutzung eines polyklonalen oder monoklonalen Antikörpers nach Anspruch 29 für die Herstellung eines Arzneimittels, dass zur Behandlung von akuter lymphoblastischer Leukämie des Typs common-B-ALL mit t(12; 21) bestimmt ist.

31. Pharmazeutische Zusammensetzung, welche als Wirkstoff einen oder mehrere polyklonale oder monoklonale Antikörper gemäß der Definition in Anspruch 29 oder 30 enthält, und einem pharmazeutisch unbedenklichen Träger.

32. Zellulärer Expressionsvektor, **dadurch gekennzeichnet, dass** er eine Nukleinsäuresequenz umfasst, welche ein immunogenes Peptid gemäß der Definition einer der Ansprüche 1 bis 16 exprimiert.

33. Vektor nach Anspruch 32, **dadurch gekennzeichnet, dass** besagte Nukleinsäuresequenz aus der Gruppe bestehend aus SEQ ID NOs: 19 bis 38 ausgewählt wird.

34. Verfahren zur Herstellung eines immunogenen Peptids gemäß der Definition einer der Ansprüche 1 bis 16, welches die Transformation eines Zellwirts mithilfe eines zellulären Expressionsvektors gemäß Anspruch 32 oder 33 umfasst, gefolgt von der Kultivierung des so transformierten Zellwirts und der Wiedergewinnung des Peptids im Nährboden.

35. Tumorale Zelle, **dadurch gekennzeichnet, dass** sie durch eine zweifache Transfektion der Maus-Zelllinie EL4S3-rob (murines β2-Mikroglobulin negativ) erzielt wird mit:
1) dem humanen Gen, welches das Molekül HHD codiert und
2) dem für das Fusionsprotein TEL/AML1 codierenden humanen Gen, das die Wildtyp-Peptidsequenz R9M enthält (SEQ ID NO: 1) oder eine mutierte Peptidsequenz R9M, welche für ein immunogenes Peptid gemäß einem der Ansprüche 1 bis 16 codiert.

36. Tumorale Zelle nach Anspruch 35, die in der nationalen Sammlung für Kulturen von Mikroorganismen CNCM des Pasteur-Instituts am 1. Dezember 2000 unter der Registrierungsnummer 1-2587 hinterlegt wurde.

37. Verfahren zur Auswahl therapeutischer Moleküle, welche *in vivo* eine schützende Immunantwort gegen tumorale, virale, bakterielle oder fungische Wildtyp-Peptide hervorrufen können, **dadurch gekennzeichnet, dass**:
1) man einem Tiermodell:
a) die zu testenden Moleküle (Gruppe 1) oder besagte tumorale, virale, bakterielle oder fungische Wildtyp-Peptide verabreicht
b) eine zweifach tumorale Zelllinie EL4S3-rob, welche von den für das Molekül HHD codierenden Nukletodisequenzen und für besagte tumorale, virale, bakterielle oder fungische Wildtyp-Peptide codierenden Nukleotidsequenzen zweifach transfektiert wurde,
wobei besagtes Tiermodell einen Genotypen aufweist, der mit dem der besagten transfektierten tumoralen Zelle kompatibel ist;
2) man die in jeder der Gruppen gegen die Epitopen besagter tumoraler, viraler, bakterieller oder fungischer Peptide hervorgerufene Immunantwort vergleicht;
3) man die Moleküle auswählt, welche *in vivo* eine stärkere Immunantwort gegen die Epitopen besagter tumoraler, viraler, bakterieller oder fungischer Peptide hervorgerufen haben, als die tumoralen, viralen, bakteriellen oder fungischen Peptide.

38. Immunogenes Polymer, welches zwei bis zehn Peptide umfasst, die ausgewählt wurden aus den immunogenen Peptiden gemäß den Definitionen der Ansprüche 1 bis 16, und den immunogenen Peptiden, welche aus SEQ ID N0s: 4 bis 7 bestehen oder diese enthalten.

39. Polynucleotid, das für eine andere Peptidsequenz als eine Wildtyp-Sequenz R9M codiert, wobei besagtes Polynucleotid folgendes inkorporiert:
- mehrere Polynucleotide, welche für die immunogenen Peptide gemäß den Definitionen in den Ansprüchen 1 bis 16 codieren; und
- mehrere Polynucleotide, deren Sequenz aus der Gruppe ausgewählt wird, die aus einer Sequenz besteht, welche aus SEQ ID NO: 19 bis 38 besteht oder diese enthält.

40. Oligonucleotid mit einer Nukleotidsequenz, welche für eine andere Peptidsequenz als eine Wildtyp-Sequenz R9M codiert, wobei besagtes Oligonukleotid eines oder mehrere Polynucleotide gemäß Anspruch 17 oder 18 inkorporiert.

41. Benutzung zytotoxischer Zellen T CD8, die aus der *in vitro*-Inkubation lymphoider Zellen stammen, die *in vitro* nach dem Verfahren des Anspruchs 27 für die Herstellung eines Arzneimittels zur Behandlung akuter lymphoblastischer Leukämie des Typs common-B-ALL mit t(12;21) hergestellt wurden.

42. Benutzung lymphoider Zellen, die *in vitro* nach dem Verfahren von Anspruch 27 zur Herstellung eines Arzneimittels für die Behandlung der akuten lymphoblastischen Leukämie des Typs common-B-ALL mit t(12;21) hergestellt wurden.

43. Mutiertes immunogenes Peptid gemäß der Definition der Ansprüche 1 bis 16 zur Benutzung bei der *in vivo*-Induktion einer zytotoxischen antileukämischen Antwort T CD8⁺.

44. Benutzung eines mutierten immunogenen Peptids gemäß der Definition der Ansprüche 1 bis 16 für die *in vitro*-Induktion einer zytotoxischen Antwort T CD8⁺.

45. Immunogenes Peptid gemäß der Definition der Ansprüche 1 bis 16 oder ein Polynucleotid gemäß den Ansprüchen 17 oder 18 zur Benutzung als Antitumormittel oder Antitumorimpfstoff.

46. Polyklonaler oder monoklonaler Antikörper nach Anspruch 29, zytotoxische Zellen T CD8, die aus der *in vitro*-Inkubation lymphoider Zellen stammen, die *in vitro* gemäß dem Verfahren von Anspruch 27 hergestellt wurden oder lymphoide Zellen, die *in vitro* gemäß dem Verfahren von Anspruch 27 hergestellt wurden, für die Benutzung bei der Behandlung akuter lymphoblastischer Leukämie des Typs common-B-ALL mit t(12;21).

## Claims

1. Immunogenic peptide, **characterised in that** it is derived from the human TEL/AML1 fusion protein comprising the wild R9M peptide sequence (SEQ ID NO: 1) and **in that** it is mutated in said R9M peptide sequence by substitution of the amino acid 1 by an aromatic amino acid, particularly by tyrosine.

2. Immunogenic peptide, **characterised in that** it is derived from the human TEL/AML1 fusion protein comprising the wild R9M peptide sequence (SEQ ID NO: 1), and **in that** it is mutated in said R9M peptide sequence by substitution of the amino acid 5 by an alpha-aminobutyric acid.

3. Immunogenic peptide, **characterised in that** it is obtained from the immunogenic peptide of claim 1, by substitution of the amino acid 5 of said R9M peptide sequence by a neutral amino acid selected from serine, alanine and alpha-aminobutyric acid.

4. Immunogenic peptide according to claim 1, **characterised in that** it consists of said R9M peptide sequence substituted in its amino acid 1 by an aromatic amino acid, particularly by tyrosine.

5. Immunogenic peptide according to claim 3, **characterised in that** it consists of said R9M peptide sequence substituted in its amino acid 1 by an aromatic amino acid, particularly by tyrosine and substituted in its amino acid 5 by a neutral amino acid selected from serine, alanine and alpha-aminobutyric acid.

6. Immunogenic peptide according to claim 2, **characterised in that** it consists of said R9M peptide sequence substituted in its amino acid 5 by an alpha-aminobutyric acid.

7. Immunogenic peptide according to claim 3 or 5, **characterised in that** it consists of a peptide sequence selected from the group consisting of SEQ ID NOs: 4, 5 and 7 or **in that** it comprises a peptide sequence selected from the group consisting of SEQ ID NOs: 4, 5, and 7.

8. Immunogenic peptide according to claim 2 or 6, **characterised in that** it consists of a peptide sequence defined by SEQ ID NO: 6 or **in that** it comprises a peptide sequence defined by SEQ ID NO: 6.

9. Immunogenic peptide according to claim 1 or 4, **characterised in that** it consists of a peptide sequence of SEQ ID NO: 43 or **in that** it comprises a peptide sequence of SEQ ID NO: 43.

10. Immunogenic peptide according to any one of claims 1 to 9, **characterised in that** it slows the growth of lymphoblastic tumour cells following administration to a leukaemic patient.

11. Immunogenic peptide according to any one of claims 1 to 10, **characterised in that** it induces an immune response against development of lymphoblastic tumour cells following administration to a recipient.

12. Immunogenic peptide according to any one of claims 1 to 11, **characterised in that**, following its administration to a leukaemic patient, it triggers an *in vivo* immune response against common B-ALL leukaemic cells with t(12;21) of said patient.

13. Immunogenic peptide according to any one of claims 1 to 11, **characterised in that** it induces an *in vitro* immune response against common B-ALL leukaemic cells with t(12;21) isolated from a leukaemic patient.

14. Immunogenic peptide according to any one of claims 1 to 11, **characterised in that** it triggers an *in vitro* cytotoxic response against common B-ALL leukaemic cells with t(12;21) isolated from a leukaemic patient.

15. Immunogenic peptide according to any one of claims 1 to 14, **characterised in that** it forms, owing to its high affinity for the HLA-A2.01 molecule, a stable complex with this molecule, making the latter capable of inducing an *in vivo* or *in vitro* cytotoxic immune response.

16. Immunogenic peptide according to claim 15, **characterised in that** said complex has a half-life of between approximately 0.9 hours and approximately 5 hours.

17. Polynucleotide encoding an immunogenic peptide according to any one of claims 1 to 16.

18. Polynucleotide of claim 17, **characterised in that** its nucleotide sequence is selected from the group consisting of the SEQ ID NOs: 19 to 38.

19. Medicinal product comprising at least one of the elements selected from the group consisting of:
a. the immunogenic peptide according to any one of claims 1 to 16;
b. the polynucleotide of claim 17 or 18; and
c. lytic T-cells sensitised *in vitro* by placing in contact an immunogenic peptide according to any one of claims 1 to 16.

20. Medicinal product according to claim 19, **characterised in that** at least a portion of the immunogenic peptides included in said medicinal product is conjugated to a carrier on to which said peptides are absorbed or bonded covalently or non-covalently.

21. Medicinal product according to claim 20, **characterised in that** the carrier is selected from the group consisting of lipid vesicles, microspheres, latex beads, polystyrene beads and proteins.

22. Medicinal product according to any one of claims 19 to 21, for therapeutic vaccination and/or preventive vaccination against leukaemia in humans.

23. Medicinal product according to claim 22, **characterised in that** the leukaemia is common B-ALL acute lymphoblastic leukaemia with t(12;21).

24. Therapeutic composition comprising a medicinal product according to any one of claims 19 to 23, and an acceptable pharmaceutical vehicle.

25. Therapeutic composition according to claim 24, for treatment or prevention of common B-cell acute lymphoblastic leukaemia with t(12;21) in humans.

26. Use of at least one immunogenic peptide selected from the peptides defined in claims 1 to 16 and/or at least one polynucleotide as defined in claim 17 or 18, for preparation of an antitumour agent or for preparation of an antitumour vaccine.

27. Method for *in vitro* stimulation of the immune response in humans, comprising separation of the human lymphoid cells and *in vitro* incubation of said cells in the presence of at least one immunogenic peptide selected from the peptides defined in claims 1 to 16 and/or in the presence of at least one polynucleotide according to claim 17 or 18, said cells allowing induction of an *in vitro* cytotoxic response.

28. Medicinal product capable of stimulating an *in vivo* immune response in humans, comprising CD8 cytotoxic T-cells obtained from *in vitro* incubation of the lymphoid cells stimulated *in vitro* according to the method of claim 27, or lymphoid cells stimulated *in vitro* according to the method of claim 27.

29. Purified polyclonal or monoclonal antibody, **characterised in that** it bonds to at least one peptide according to any one of claims 1 to 16.

30. Use of a polyclonal or monoclonal antibody according to claim 29 to prepare a medicinal product intended for treatment of common B-ALL acute lymphoblastic leukaemia with t(12;21).

31. Pharmaceutical composition comprising by way of its active substance one or several polyclonal or monoclonal antibody or antibodies as defined in claim 29 or 30 with an acceptable pharmaceutical vehicle.

32. Cell expression vector, **characterised in that** it comprises a nucleic acid sequence expressing an immunogenic peptide as defined by any one of claims 1 to 16.

33. Vector of claim 32, **characterised in that** said nucleic acid sequence is selected from the group consisting of the SEQ ID NOs: 19 to 38.

34. Method of preparing an immunogenic peptide as defined according to any one of claims 1 to 16, comprising transformation of a cell host by means of a cell expression vector as defined in claim 32 or 33, followed by culturing the cell host thus transformed, and recovering the peptide in the culture medium.

35. Tumour cell **characterised in that** it is obtained by double transfection of EL4 S3-Rob (murine β2-microglobulin negative) mice with:
1) the human gene encoding the HHD molecule; and
2) the human gene encoding the TEL/AML1 fusion protein comprising the wild R9M peptide sequence (SEQ ID NO: 1); or a mutated R9M peptide sequence encoding a mutated immunogenic peptide as defined according to any one of claims 1 to 16.

36. Tumour cell according to claim 35, deposited in the Institute Pasteur National Microorganism Culture Collection (CNCM) on 1st December 2000 under registration number I-2587.

37. Method for selecting therapeutic molecules capable of inducing an *in vivo* protective immune response against tumoural, viral, bacterial or fungal wild-type peptides, **characterised in that**:
1) the following are administered in an animal model:
a) the molecules to be tested (group 1) or said tumoural, viral, bacterial or fungal wild-type peptides
b) an EL4S3-rob tumour cell line doubly transfected with nucleotide sequences encoding the HHD molecule and nucleotide sequences encoding said tumoural, viral, bacterial or fungal wild-type peptides,
wherein said animal model has a genotype compatible with that of said transfected tumour cell;
2) the immune response against the epitopes of said tumoural, viral, bacterial or fungal peptides induced in each of the groups is compared;
3) the molecules are selected that induced an *in vivo* immune response against the epitopes of said tumoural, viral, bacterial or fungal peptides greater than that induced by said wild-type tumoural, viral, bacterial or fungal peptides.

38. Immunogenic polymer comprising between two and ten peptides selected from consisting of the immunogenic peptides as defined in claims 1 to 16 and the immunogenic peptides consisting of or comprising SEQ ID NOs: 4 to 7.

39. Polynucleotide encoding a peptide sequence other than a wild-type R9M sequence, wherein said polynucleotide incorporates:
- several polynucleotides encoding immunogenic peptides such as defined in claims 1 to 16; and
- several polynucleotides, the sequence of which is selected from the group consisting of a sequence comprising or consisting of SEQ ID NO: 19 to 38.

40. Oligonucleotide having a nucleotide sequence encoding a peptide sequence other than a wild-type R9M sequence, wherein said oligonucleotide incorporates one or several polynucleotides according to claim 17 or 18.

41. Use of cytotoxic CD8 T-cells obtained from *in vitro* incubation of lymphoid cells prepared *in vitro* according to the method of claim 27 to prepare a medicinal product intended to treat common B-ALL acute lymphoblastic leukaemia with t(12;21).

42. Use of lymphoid cells prepared *in vitro* according to the method of claim 27 to prepare a medicinal product intended for treatment of common B-ALL acute lymphoblastic leukaemia with t(12;21).

43. Mutated immunogenic peptide as defined in claims 1 to 16 for use in the *in vivo* induction of an antileukaemic CD8+ cytotoxic T-cell response.

44. Use of a mutated immunogenic peptide as defined in claims 1 to 16 for *in vitro* induction of a CD8+ cytotoxic T-cell response.

45. Immunogenic peptide as defined in claims 1 to 16 or polynucleotide as defined in claim 17 or 18 for use as an antitumour agent or antitumour vaccine.

46. Polyclonal or monoclonal antibody according to claim 29, cytotoxic CD8 T-cells obtained from *in vitro* incubation of lymphoid cells prepared *in vitro* according to the method of claim 27 or lymphoid cells prepared *in vitro* according to the method of claim 27 for use in the treatment of common B-ALL acute lymphoblastic leukaemia with t(12;21).


### SEQUENCE LISTING

<110> Institut Pasteur
   Institut National de la Santé et de la Recherche Médicale (INSERM) Assistance Publique-Hôpitaux de Paris
<120> MUTATED IMMUNOGENIC PEPTIDES DERIVED FROM R9M, POLYNUCLEOTIDES CODING FOR
   THE SAME AND THEIR THERAPEUTIC USES
<130> EGYP - B5098A
<150> CA 2,325,666
   <151> 2000-12-01
<160> 45
<170> PatentIn version 3.1
<210> 1
   <211> 9
   <212> PRT
   <213> Homo sapiens
<400> 1
   Arg Ile Ala Glu Cys Ile Leu Gly Met
   1               5
<210> 2
   <211> 9
   <212> PRT
   <213> Artificial sequence
<220>
   <223> sequence is completely synthesized
<400> 2
   Arg Ile Ala Glu Ser Ile Leu Gly Met
   1               5
<210> 3
   <211> 9
   <212> PRT
   <213> Artificial sequence
<220>
   <223> sequence is completely synthesized
<400> 3
   Arg Ile Ala Glu Ala Ile Leu Gly Met
   1               5
<210> 4
   <211> 9
   <212> PRT
   <213> Artificial sequence
<220>
   <223> sequence is completely synthesized
<400> 4
   Tyr Ile Ala Glu Ser Ile Leu Gly Met
   1               5
<210> 5
   <211> 9
   <212> PRT
   <213> artificial sequence
<220>
   <223> sequence is completely synthesized
<400> 5
   Tyr Ile Ala Glu Ala Ile Leu Gly Met
   1               5
<210> 6
   <211> 9
   <212> PRT
   <213> artificial sequence
<220>
   <223> sequence is completely synthesized
<220>
   <221> MISC_FEATURE
   <222> (5)..(5)
   <223> X is an alpha-aminobutyric acid fixed to a glutamic acid
<400> 6
   Arg Ile Ala Glu Xaa Ile Leu Gly Met
   1               5
<210> 7
   <211> 9
   <212> PRT
   <213> artificial sequence
<220>
   <223> sequence is completely synthesized
<220>
   <221> MISC_FEATURE
   <222> (5).. (5)
   <223> X is an alpha-aminobutyric acid fixed to a glutamic acid
<400> 7
   Tyr Ile Ala Glu Xaa Ile Leu Gly Met
   1               5
<210> 8
   <211> 27
   <212> DNA
   <213> Homo sapiens
<400> 8
   agaatagcag aatgcatact tggaatg 27
<210> 9
   <211> 27
   <212> DNA
   <213> artificial sequence
<220>
   <223> sequence is completely synthesized
<400> 9
   agaatagcag aaagcatact tggaatg 27
<210> 10
   <211> 27
   <212> DNA
   <213> artificial sequence
<220>
   <223> sequence is completely synthesized
<400> 10
   agaatagcag aaagtatact tggaatg 27
<210> 11
   <211> 27
   <212> DNA
   <213> artificial sequence
<220>
   <223> sequence is completely synthesized
<400> 11
   agaatagcag aatcaatact tggaatg 27
<210> 12
   <211> 27
   <212> DNA
   <213> artificial sequence
<220>
   <223> sequence is completely synthesized
<400> 12
   agaatagcag aatccatact tggaatg 27
<210> 13
   <211> 27
   <212> DNA
   <213> artificial sequence
<220>
   <223> sequence is completely synthesized
<400> 13
   agaatagcag aatcgatact tggaatg 27
<210> 14
   <211> 27
   <212> DNA
   <213> artificial sequence
<220>
   <223> sequence is completely synthesized
<400> 14
   agaatagcag aatctatact tggaatg 27
<210> 15
   <211> 27
   <212> DNA
   <213> artificial sequence
<220>
   <223> sequence is completely synthesized
<400> 15
   agaatagcag aagcaatact tggaatg 27
<210> 16
   <211> 27
   <212> DNA
   <213> artificial sequence
<220>
   <223> sequence is completely synthesized
<400> 16
   agaatagcag aagccatact tggaatg 27
<210> 17
   <211> 27
   <212> DNA
   <213> artificial sequence
<220>
   <223> sequence is completely synthesized
<400> 17
   agaatagcag aagcgatact tggaatg 27
<210> 18
   <211> 27
   <212> DNA
   <213> artificial sequence
<220>
   <223> sequence is completely synthesized
<400> 18
   agaatagcag aagctatact tggaatg 27
<210> 19
   <211> 27
   <212> DNA
   <213> artificial sequence
<220>
   <223> sequence is completely synthesized
<400> 19
   tacatagcag aaagcatact tggaatg 27
<210> 20
   <211> 27
   <212> DNA
   <213> artificial sequence
<220>
   <223> sequence is completely synthesized
<400> 20
   tatatagcag aaagcatact tggaatg 27
<210> 21
   <211> 27
   <212> DNA
   <213> artificial sequence
<220>
   <223> sequence is completely synthesized
<400> 21
   tacatagcag aaagtatact tggaatg 27
<210> 22
   <211> 27
   <212> DNA
   <213> artificial sequence
<220>
   <223> sequence is completely synthesized
<400> 22
   tatatagcag aaagtatact tggaatg 27
<210> 23
   <211> 27
   <212> DNA
   <213> artificial sequence
<220>
   <223> sequence is completely synthesized
<400> 23
   tacatagcag aatcaatact tggaatg 27
<210> 24
   <211> 27
   <212> DNA
   <213> artificial sequence
<220>
   <223> sequence is completely synthesized
<400> 24
   tatatagcag aatcaatact tggaatg 27
<210> 25
   <211> 27
   <212> DNA
   <213> artificial sequence
<220>
   <223> sequence is completely synthesized
<400> 25
   tacatagcag aatccatact tggaatg 27
<210> 26
   <211> 27
   <212> DNA
   <213> artificial sequence
<220>
   <223> sequence is completely synthesized
<400> 26
   tatatagcag aatccatact tggaatg 27
<210> 27
   <211> 27
   <212> DNA
   <213> artificial sequence
<220>
   <223> sequence is completely synthesized
<400> 27
   tacatagcag aatcgatact tggaatg 27
<210> 28
   <211> 27
   <212> DNA
   <213> artificial sequence
<220>
   <223> sequence is completely synthesized
<400> 28
   tatatagcag aatcgatact tggaatg 27
<210> 29
   <211> 27
   <212> DNA
   <213> artificial sequence
<220>
   <223> sequence is completely synthesized
<400> 29
   tacatagcag aatctatact tggaatg 27
<210> 30
   <211> 27
   <212> DNA
   <213> artificial sequence
<220>
   <223> sequence is completely synthesized
<400> 30
   tatatagcag aatctatact tggaatg 27
<210> 31
   <211> 27
   <212> DNA
   <213> artificial sequence
<220>
   <223> sequence is completely synthesized
<400> 31
   tacatagcag aagcaatact tggaatg 27
<210> 32
   <211> 27
   <212> DNA
   <213> artificial sequence
<220>
   <223> sequence is completely synthesized
<400> 32
   tatatagcag aagcaatact tggaatg 27
<210> 33
   <211> 27
   <212> DNA
   <213> artificial sequence
<220>
   <223> sequence is completely synthesized
<400> 33
   tacatagcag aagccatact tggaatg 27
<210> 34
   <211> 27
   <212> DNA
   <213> artificial sequence
<220>
   <223> sequence is completely synthesized
<400> 34
   tatatagcag aagccatact tggaatg 27
<210> 35
   <211> 27
   <212> DNA
   <213> artificial sequence
<220>
   <223> sequence is completely synthesized
<400> 35
   tacatagcag aagcgatact tggaatg 27
<210> 36
   <211> 27
   <212> DNA
   <213> artificial sequence
<220>
   <223> sequence is completely synthesized
<400> 36
   tatatagcag aagcgatact tggaatg 27
<210> 37
   <211> 27
   <212> DNA
   <213> artificial sequence
<220>
   <223> sequence is completely synthesized
<400> 37
   tacatagcag aagctatact tggaatg 27
<210> 38
   <211> 27
   <212> DNA
   <213> artificial sequence
<220>
   <223> sequence is completely synthesized
<400> 38
   tatatagcag aagctatact tggaatg 27
<210> 39
   <211> 9
   <212> PRT
   <213> artificial sequence
<220>
   <223> sequence is completely synthesized
<400> 39
   Ala Met Pro Ile Gly Arg Ile Ala Glu
   1               5
<210> 40
   <211> 9
   <212> PRT
   <213> artificial sequence
<220>
   <223> sequence is completely synthesized
<400> 40
   Tyr Met Pro Ile Gly Arg Ile Ala Glu
   1               5
<210> 41
   <211> 9
   <212> PRT
   <213> artificial sequence
<220>
   <223> sequence is completely synthesized
<400> 41
   Pro Ile Gly Arg Ile Ala Glu Cys Ile
   1               5
<210> 42
   <211> 9
   <212> PRT
   <213> artificial sequence
<220>
   <223> sequence is completely synthesized
<400> 42
   Tyr Ile Gly Arg Ile Ala Glu Cys Ile
   1 5
<210> 43
   <211> 9
   <212> PRT
   <213> artificial sequence
<220>
   <223> sequence is completely synthesized
<400> 43
   Tyr Ile Ala Glu Cys Ile Leu Gly Met
   1 5
<210> 44
   <211> 9
   <212> PRT
   <213> Human immunodeficiency virus
<400> 44
   Ile Leu Lys Glu Pro Val His Gly Val
   1 5
<210> 45
   <211> 10
   <212> PRT
   <213> human herpesvirus
<400> 45
   Phe Leu Pro Ser Asp Tyr Phe Pro Ser Val
   1 5 10